# DEMANDE DE BREVET EUROPEEN

(11) **EP 4 060 021 A1**
(43) Date de publication de la demande: **21.09.2022**
(21) Numéro de dépôt: 21305337.4
(22) Date de dépôt: 18.03.2021
(51) Int. Cl.: C12N 1/20, C12Q 1/18

(54) **MILIEU MIMETIQUE DE CULTURE MICROBIOLOGIQUE ET UTILISATIONS DE CELUI-CI**

(71) Demandeur: BIOFILM PHARMA, 69007 Lyon (FR); Institut National de la Santé et de la Recherche Médicale (INSERM), 75013 Paris (FR); UNIVERSITE DE MONTPELLIER, 34090 Montpellier (FR)
(72) Inventeur: POUGET, Cassandra, 34400 LUNEL (FR); LAVIGNE, Jean-Philippe, 30820 CAVEIRAC (FR); DUNYACH-REMY, Catherine, 34070 MONTPELLIER (FR); SOTTO, Albert, 30900 NIMES (FR); PROVOT, Christian, 63670 LE CENDRE (FR); TASSE, Jason, 69008 LYON (FR); BERNARDI, Thierry, 63170 PERIGNAT-LES-SARLIEVE (FR)
(74) Mandataire: Novagraaf Technologies

(57) **Abrégé**

La présente invention se rapporte à un milieu de culture de microorganismes comprenant un hydrolysat enzymatique de protéines, de préférence un hydrolysat pepsique de protéines, un hydrolysat de protéines, un extrait de levure, du sérum de mammifère, du sang de mammifère, des débris cellulaires et un sel.

La présente invention se rapporte également à l'utilisation du milieu de culture dans un procédé de formation d'un biofilm, dans un procédé de détermination de la sensibilité de microorganismes à des composés, et dans un procédé de culture d'une pluralité de microorganismes.

La présente invention se rapporte également à un procédé de fabrication d'un milieu de culture de microorganismes.

La présente invention trouve une application notamment dans le domaine pharmaceutique, le domaine médical, le domaine vétérinaire, le domaine cosmétique.

## Description

### Domaine technique

La présente invention se rapporte à un milieu de culture de microorganismes comprenant un hydrolysat enzymatique de protéines, un hydrolysat de protéines, un extrait de levure, du sérum de mammifère décomplémenté, du sang de mammifère, des débris cellulaires et au moins un sel, dans lequel le pH du milieu de culture est compris entre 7,5 et 8,5.

La présente invention se rapporte également à l'utilisation du milieu de culture dans un procédé de détermination de la sensibilité de microorganismes à des composés.

La présente invention se rapporte également à l'utilisation du milieu de culture dans un procédé de détermination et/ou d'identification de la formation d'un biofilm, en particulier par des microorganismes.

La présente invention se rapporte également à l'utilisation du milieu de culture dans un procédé de culture de microorganismes isolés ou d'une pluralité de microorganismes.

La présente invention se rapporte également un procédé de fabrication d'un milieu de culture de microorganismes.

La présente invention trouve une application notamment dans le domaine pharmaceutique, le domaine médical, le domaine vétérinaire, le domaine analytique, le domaine cosmétique.

Dans la description ci-dessous, les références entre crochets ([]) renvoient à la liste des références présentée à la fin du texte.

### Etat de la technique

### Les biofilms et les plaies

Les bactéries peuvent exister sous forme libre (dite forme planctonique) ou attachées à un support (dite forme sessile), formant un biofilm. Alors que la plupart des pathologies infectieuses sont associées à des bactéries sous forme planctonique (exemple du sepsis), d'autres sont liées à des bactéries sous forme biofilm. Cet état, qui modifie les caractéristiques bactériennes, devrait être pris en compte notamment lors de l'évaluation *in vitro* des sensibilités aux antimicrobiens. D'après Donlan et Costerton (2002) [1] et Høiby (2014) [2], le biofilm est une communauté sessile et polymicrobienne caractérisée par des bactéries qui, soit s'attachent irréversiblement à une surface ou une interface, soit forment des agrégats qui peuvent ne pas être directement adhérents sur une surface ou une interface. Ces bactéries sont agglomérées dans une « protection » qu'elles produisent : l'EPS (Extracellular Polymeric Substance). La présence à l'intérieur d'un biofilm permet aux bactéries de modifier leur comportement en termes, entre autres, de croissance et d'expression génique (Liu 1999) [3]. Le biofilm est présent dans de nombreuses maladies infectieuses comme l'endocardite, la prostatite, la périodontite, les infections du pied chez le diabétique, la surinfection dans le cas de mucoviscidose, l'otite moyenne et est observé sur certains dispositifs médicaux comme les prothèses ou cathéters par exemple. Potera (1999) [4] a rapporté que 65% des infections humaines induisaient du biofilm (Potera 1999) [4]. Cependant, la présence de biofilm n'est pas nécessairement délétère. Le biofilm est produit naturellement au niveau des dents, du tube digestif ou de la muqueuse vaginale, formant une protection contre les bactéries pathogènes (Reid, Howard & Gan 2001) [5].

Dans les plaies chroniques, l'organisation en biofilm est très classique. Elle est observée dans les ostéites du pied (Gristina et al. 1985) [6] et est une complication fréquente des plaies chez les patients diabétiques (Newman et al. 1991) [7]. James et al. ont rapporté que 60% des plaies chroniques présentaient du biofilm contre 6% des plaies aigues (James et al. 2008) [8]. A noter que 13 des 50 plaies chroniques analysées étaient des plaies du pied de patients diabétiques et 10 (77%) contenaient du biofilm. La conséquence de ces biofilms au niveau des plaies chroniques est un retard de cicatrisation de la plaie et une chronicisation de l'infection (Metcalf, 2013) [9].

Il est depuis longtemps admis qu'une plaie présente un exsudat dont la fonction principale est de prévenir la dessiccation qui conduirait à un retard de cicatrisation (Hinman, 1963) [10]. Une plaie aigue contient des protéases dont le rôle est de préparer le lit de la plaie et de dégrader les composants de la matrice extracellulaire, telles que le collagène et l'élastine, afin de préparer le processus de re-épithalisation de la plaie (Cutting, 2003) [11]. Elle contient également les éléments suivants (Cutting, 2003) : sodium, potassium, chlorure, urée, créatinine, en concentrations similaires à celles retrouvées dans le sérum, bicarbonate, cytokines (Interleukines, TNF-β, Interféron-a), leucocytes, polynucléaires neutrophiles, monocytes/macrophage, débris de cellules, lysozyme, protéines (à un taux plus faible que celui du sérum), Metallo-Protéinases matricielles (Matrix MetalloProteinase ; MMP). Dans le cas d'une plaie chronique, les composants sont similaires, la différence portant essentiellement sur le pH qui reste alcalin et le taux de métalloprotéinases qui reste élevé dans le temps.

Les plaies constituent des environnements particuliers qui, pour être étudiés et/ou pour étudier l'effet de molécules, médicaments et/ou agents pathogènes dans/sur ces environnements nécessitent l'obtention de modèle de plaies. Toutefois, il n'existe pas actuellement de modèle de référence de plaies chroniques et/ou non chroniques permettant, notamment l'étude de l'effet de molécules, médicaments et/ou agents pathogènes. En outre, il n'existe pas actuellement de modèle fiable et/ou représentatif de plaies chroniques et/ou non chroniques.

Aussi, il existe un réel besoin de trouver un nouveau moyen/composé palliant ces défauts, inconvénients, faiblesses et obstacles de l'art antérieur, en particulier d'un moyen/milieu représentatif et/ou similaire à celui d'une plaie permettant leur étude. Il existe également un réel besoin de trouver un modèle fiable, et permettant l'obtention de résultats reproductibles et comparables.

Il existe dans l'état de la technique des milieux de culture pour les microorganismes, en particulier pour les bactéries. Classiquement, en laboratoire de bactériologie, les bactéries sont cultivées dans des milieux standardisés adaptés à la croissance générale de microorganismes, ou plus spécifiquement à la croissance de certains microorganismes (milieux sélectifs). Certains de ces milieux sont couramment utilisés pour l'évaluation de la sensibilité de microorganismes à des composés antimicrobiens. Les conditions d'utilisation pour l'étude de la sensibilité des microorganismes aux antimicrobiens ont été standardisées en 1961, et les tests pratiqués s'appuient toujours sur ces recommandations (WHO, 1961) [12]. Le point clé qui guide une décision concernant une antibiothérapie est la Concentration Critique (« clinical breakpoint ») d'un antibiotique qui est revue régulièrement en France et en Europe par le Comité de l'Antibiogramme de la Société Française de Microbiologie (CASFM) / EUCAST (European Committee on Antimicrobial Susceptibility Testing) ou aux USA par le CLSI (Clinical and Laboratory Standards Institute). Ces concentrations critiques sont établies selon une procédure séquentielle prenant en compte 4 paramètres : (1) l'efficacité *in vitro* d'un antibiotique, déterminée par une procédure standard de susceptibilité (AST ; Antibiotic Susceptibility Testing) ; (2) les paramètres de pharmacocinétique / pharmacodynamique (PK/PD) chez l'animal ; (3) l'efficacité / toxicité établie chez l'animal pour des pathogènes modèles, et (4) les protocoles de dosage validés sur un nombre limité de données cliniques. Bien que l'efficacité *in vitro,* en tests AST, ne représente qu'une partie de cette procédure, elle représente le premier test, et sa pertinence physiologique de par l'utilisation du milieu particulier Mueller-Hinton (MH) est importante. En particulier ce milieu, qui est considéré comme un milieu « riche » adapté à la croissance de microorganismes, en particulier de bactéries, ne reproduit pas les éventuelles conditions particulières physiologiques rencontrées par exemple sur les muqueuses, sur la peau, etc.

Aussi, il existe un réel besoin de trouver un nouveau moyen/milieu palliant ces défauts, inconvénients, faiblesses et obstacles de l'art antérieur, en particulier d'un moyen/milieu représentatif et/ou similaire aux conditions physiologiques rencontrées, par exemple au niveau du revêtement et/ou de la surface cutané, notamment au cours de plaies, et des muqueuses.

Il existe dans l'état de la technique différents milieux utilisés pour la croissance des microorganismes, en particulier le milieu MH (très utilisé pour les tests de sensibilité de microorganismes aux antimicrobiens ; AST), le Bouillon de Soja Tryptique « Tryptic Soy Broth » (TSB), le « Lysogeny broth » (LB) ou le Bouillon Cœur Cervelle (« Brain Heart Infusion » (BHI)). La composition de ces différents milieux est :
- MH (Mueller Hinton) pour 1 litre :
   ∘ Extrait de viande (bœuf) 2,0 g
   ∘ Hydrolysat de caséines 17,5 g
   ∘ Amidon 1,5 g
   ∘ pH 7,2 - 7,6 à 25°C
      éventuellement supplémenté en chlorure de calcium, en chlorure de magnésium, ou en sang
   ∘ CaCl₂ 20-25 mg/L
   ∘ MgCl₂ 10-12,5 mg/L
   ∘ 5-10% de sang de cheval défibriné ou 5% de sang de mouton
- TSB (Tryptic Soy Broth ; Bouillon de Soja Tryptique) pour 1 litre :
   ∘ Hydrolysat de caséines 17,0 g
   ∘ Digestion peptique de semoule de soja 3,0 g
   ∘ Glucose 2,5 g
   ∘ Chlorure de sodium 5,0 g
   ∘ Hydrogénophosphate de potassium 2,5 g
   ∘ pH 7,1 - 7,5 à 25°C
- LB (Lysogeny broth) pour 1 litre :
   ∘ Tryptone 10,0 g
   ∘ Extrait de levure 5,0 g
   ∘ NaCl 10,0 g
   ∘ pH 7,0
- BHI (Brain Heart Infusion ; Bouillon Cœur Cervelle) pour 1 litre :
   ∘ Protéose-peptone 10,0 g
   ∘ Infusion de cervelle de veau 12,5 g
   ∘ Infusion de cœur de bœuf 5,0 g
   ∘ Glucose 2,0 g
   ∘ Chlorure de sodium 5,0 g
   ∘ Hydrogénophosphate de sodium 2,5 g
   ∘ pH = 7,2 - 7,6 à 25°C

L'ensemble des milieux précités sont des milieux de croissance bactérienne, dits « riches » adaptés à une bonne croissance bactérienne. En d'autres termes, les milieux précités ont pour but d'assurer une croissance optimale des microorganismes, en particulier des bactéries.

Les milieux précités ne sont aucunement représentatifs d'un quelconque environnement physiologique, par exemple de la peau, de muqueuses, etc.

Aussi, il existe un réel besoin de trouver un nouveau moyen/milieu palliant ces défauts, inconvénients et obstacles de l'art antérieur, en particulier d'un moyen/milieu représentatif et/ou similaire aux conditions physiologiques rencontrées, par exemple au niveau du revêtement cutané et/ou de la surface cutanée.

L'influence du milieu sur les tests de susceptibilité bactérienne aux antimicrobiens, devant permettre de définir à quel antibiotique, et à quelle concentration, une bactérie est sensible, et par voie de conséquence contribuer au choix d'une antibiothérapie adaptée, est assez discutée, et depuis longtemps. Une étude collaborative internationale a comparé l'efficacité d'antimicrobiens, sur le principe de tests de diffusion de 9 antibiotiques, sur 6 souches bactériennes, sur 4 milieux gélosés classiquement utilisés : TSB (Trypticase Soy), Grove & Randall formule 9, MH (Mueller-Hinton) avec ou sans L-tryptophane à 1mg/L. Des différences significatives entre les diamètres d'inhibition de croissance ont été observées pour la plupart des antimicrobiens en fonction des milieux utilisés. Ces différences peuvent avoir comme conséquences possibles de qualifier un antibiotique inefficace alors qu'il pourrait l'être, ou, plus grave, de qualifier un antibiotique efficace alors qu'il ne l'est pas.

Aussi, il existe un réel besoin de trouver un nouveau moyen/milieu palliant ces défauts, inconvénients et obstacles de l'art antérieur, en particulier d'un moyen/milieu représentatif et/ou similaire aux conditions physiologiques rencontrées, par exemple au niveau du revêtement cutané lésé et/ou d'une lésion cutanée afin de permettre l'obtention et la réalisation de tests, par exemple d'antibiogramme plus fiable.

En outre, il existe un réel besoin de trouver un nouveau moyen/milieu permettant avantageusement une classification plus précise et/ou fiable de composés, et/ou une caractérisation plus fiable de l'effet pharmacologique d'un composé, par exemple de l'efficacité antibiotique éventuelle d'un composé.

Il est également connu que l'influence du milieu n'affecte pas seulement les tests de sensibilité bactériens, mais aussi les tests de sensibilité d'autres microorganismes, par exemple fongiques. Pour certains microorganismes, il a été montré que l'utilisation de milieux MH ou TSB conduisait à déterminer des Concentrations Minimales Inhibitrices (CMI) plus faibles que dans des milieux tels que le Nutrient Broth ou le YDB (Yeast Dextrose Broth). Par exemple, la CMI (Concentration Minimale Inhibitrice) de *Saccharomyces cerevisiae* en MH est 4 fois inférieure à celle observée en YDB (Sawer, 1997 [13]).

Aussi, il existe un réel besoin de trouver un nouveau moyen/milieu palliant ces défauts, inconvénients et obstacles de l'art antérieur, en particulier d'un moyen/milieu permettant l'obtention et la réalisation de tests, par exemple de sensibilité de microorganismes à des composés plus fiables et/ou reproductibles.

En particulier, il a été démontré sur des souches cliniques de staphylocoques, et en particulier des souches de *S. aureus* issues de plaies (Ersoy, 2017 [14]) que, comparativement à la sensibilité établie en milieu MH, la croissance de souches de *S. aureus* dans du milieu de culture cellulaire DMEM (Dulbecco's Modified Eagle Médium) ou du milieu Lacks modifié (MLM ; simulant le nasopharynx) accroit la sensibilité des souches d'un facteur 4 à 256 (CMI plus basses) pour l'azithromycine, l'érythromycine, la streptomycine, le ceftriaxone et la cefalotine. Au contraire, la résistance de ces souches à la daptomycine et la rifampicine est augmentée en milieu MLM d'un facteur 4 à 16 (CMI plus hautes). Dans le cas de la daptomycine et la rifampicine, les souches étant considérées comme sensibles en milieu MH, ces 2 antibiotiques auraient donc pu être prescrits en antibiothérapies, alors qu'ils sont considérés comme inefficaces dans la condition MLM. Il apparait donc clairement que le milieu de culture a un effet sur la susceptibilité des microorganismes (notamment des bactéries) aux antibiotiques et peut induire des résultats totalement contradictoires qui peuvent être préjudiciables aux patients en générant des résultats faussement positifs ou faussement négatifs.

De plus, une étude *in vivo* utilisant un modèle de sepsis chez la souris infectée par une souche de *S. aureus* a été réalisée pour évaluer ces différences d'activité des antibiotiques en fonction du milieu de culture. Les souris traitées avec de la cefalotine, du ceftriaxone et de l'érythromycine, considérés comme inefficaces en milieu MH, mais efficaces en DMEM ou MLM, ont survécu, démontrant que ces antibiotiques étaient efficaces chez la souris et auraient pu être utilisés en antibiothérapie. A l'inverse, les souris traitées par le co-trimoxazole, qualifié comme inactif par les auteurs, alors qu'il est considéré comme efficace en milieu MH, n'a pas permis la survie des souris.

Par ailleurs, pour la daptomycine, les souches de *S. aureus* sont qualifiées de plus résistantes en milieux MLM ou Lacks, alors qu'elles sont plus sensibles en milieu DMEM.

Ces éléments démontrent clairement que les milieux utilisés en routine sur de nombreux microorganismes ne permettent pas d'obtenir des résultats homogènes.

Aussi, il existe un réel besoin de trouver un nouveau moyen/milieu palliant ces défauts, inconvénients et obstacles de l'art antérieur, en particulier d'un moyen/milieu permettant la prise en compte des facteurs environnementaux normalement présents à l'échelle des interactions hôte-pathogènes.

Il existe également un réel besoin de trouver un milieu / moyen similaire et/ou mimant l'environnement infectieux.

En outre, il existe un réel besoin de trouver un nouveau moyen/milieu palliant ces défauts, inconvénients et obstacles de l'art antérieur, en particulier d'un moyen/milieu permettant la prise en compte des facteurs environnementaux et une détermination de la sensibilité d'un microorganisme, notamment d'une bactérie, à des composés, notamment aux antimicrobiens, plus fiable.

D'autre part, il est également connu que des affections/infections, par exemple de plaies, peuvent être plurimicrobiennes. Aussi, lorsque plusieurs microorganismes sont présents lors d'une infection / affection, des interactions entre les microorganismes, par exemple entre des souches bactériennes existent et peuvent altérer ou faciliter leur croissance, la formation de biofilm, etc.

Il existe donc un réel besoin de trouver un nouveau moyen/milieu permettant une coculture de microorganismes.

Il existe également un réel besoin de trouver un nouveau moyen/milieu permettant une coculture de microorganismes tout en permettant la prise en compte des facteurs environnementaux et/ou une détermination de la sensibilité d'un microorganisme, notamment d'une bactérie, à des composés, notamment aux antimicrobiens, plus fiable.

Par ailleurs, il est connu que les microorganismes, par exemple les bactéries, présentent différentes formes de croissance et/ou peuvent être testées dans différentes conditions, par exemple sous forme planctoniques (en suspension dans le milieu), ou sous forme biofilm (attachées sur un support biotique ou abiotique, et structurées au sein d'une matrice extracellulaire).

Il existe donc un réel besoin de trouver un nouveau moyen/milieu permettant également une culture de microorganismes dans différentes conditions, par exemple pour des bactéries sous forme planctoniques ou sous forme biofilm.

Il existe également un réel besoin de trouver un nouveau moyen/milieu permettant de tester la croissance de microorganismes, par exemple de bactéries quel que soit le mode de culture, et également de tester l'effet de composés/agents physiques ou chimiques, par exemple des antimicrobiens.

Il existe des modèles ayant été développés pour étudier les biofilms multi-espèces observés dans les plaies chroniques, en particulier la formation de biofilm par 3 microorganismes isolés dans les plaies chroniques, à savoir *Staphylococcus aureus, Pseudomonas aeruginosa* et *Enterococcus faecalis* (Sun, 2008 [15]). Il s'agit notamment du milieu Lubbock basé sur le milieu de Bolton (Pitts, 2003 [16]) comprenant en outre 50% de plasma défibriné de cheval ainsi que 5% d'hématies de cheval hémolysées. Ce milieu a notamment été utilisé afin de montrer d'éventuelles interactions synergiques entre *S. aureus* et *P. aeruginosa* (DeLeon, 2014 [17]). Les auteurs ont montré que la coculture de *S. aureus* avec *P. aeruginosa* dans du milieu Lubbock est possible. Il a été montré que ladite coculture dans du milieu Lubbock permet une résistance partielle de la souche *S. aureus* à la gentamicine, ce qui n'est pas le cas de *P. aeruginosa.*

Toutefois, le milieu Lubbock ne tient pas compte d'un certain nombre de facteurs environnementaux. En outre, les milieux connus ont été établis à partir de constituants éloignés et/ou très différents de ceux trouvés, par exemple dans des plaies humaines, par exemple du plasma de bovin et du sang de cheval ont été utilisés, et un pH de l'ordre de 7,2 - 7,4. Comme mentionné ci-dessus, la composition du milieu de culture influe clairement les résultats obtenus lors d'évaluation de la sensibilité des bactéries aux antibiotiques. En outre, les milieux connus ne reproduisent pas les conditions physiologiques et/ou environnementales et/ou facteurs environnementaux, par exemple de plaies pour lesquelles les cellules sont en contact avec le sérum (Bandyopadhyay, 2006 [18]). Cette différence impacte le processus de cicatrisation et également le comportement des bactéries : croissance, sensibilité aux antimicrobiens, sous forme planctonique ou biofilm. Par ailleurs, il a été montré (Loesche, 2016 [19], Jneid, 2018 [20]) que les interactions bactériennes existant au niveau des plaies chroniques ont un impact important sur l'évolution de la plaie et peuvent, soit être bénéfiques pour la plaie en termes de cicatrisation, soit avoir un impact négatif en favorisant le retard de cicatrisation de la plaie et potentiellement le passage au stade infectieux.

Un milieu IVWM (« In Vitro Wound Milieu ») a été proposé par Kadam et al (2021) pour étudier spécifiquement les biofilms de plaies [21]. Ce milieu IVWM est constitué de sérum de veau fœtal (SVF; 70%), acide lactique (11-12mM), lactoferrine (20-30µg/mL), fibrinogène (200-400µg/mL), fibronectine (30-60µg/mL), collagène (10-12µg/mL), et est ajusté à pH 5,25, ce pH acide étant celui d'une peau saine, et non celui d'une plaie plus alcalin. Les auteurs ont comparé la croissance d'une souche de *S. aureus* AH133, d'origine non précisée et d'une souche de référence *P. aeruginosa* (PAO1) non clinique, seule ou en co-culture. La croissance en milieu IVWM des souches *S. aureus* et *P. aeruginosa* était moins rapide qu'en milieu LB, surtout celle de *S. aureus.* En co-culture, autant en LB qu'en IVWM, la croissance de *S. aureus* a été fortement diminuée. La mesure de sensibilité aux antibiotiques, en particulier la tobramycine pour *P. aeruginosa* et vancomycine pour *S. aureus)* a été faite sur des bactéries dans des biofilms établis et non sur des bactéries en état planctonique. Ce milieu a ainsi été décrit pour mesurer la croissance bactérienne de *S. aureus* et *P. aeruginosa* en culture simple ou coculture ainsi que mesurer une sensibilité de bactéries particulières dans un biofilm établi vis-à-vis d'un antibiotique (1 antibiotique testé sur chacune des 2 souches). Toutefois, ce milieu n'est pas utilisé pour la détermination de la CMI et/ou ne permet pas une utilisation et/ou n'apporte pas un quelconque effet dans la détermination de la CMI de souches planctoniques, comme cela est classiquement réalisé en routine dans les laboratoires de microbiologie médicale.

Il existe donc un réel besoin de trouver un nouveau moyen/milieu permettant une coculture ou monoculture de microorganismes, notamment dans différentes conditions, par exemple planctonique ou biofilm, et/ou de tester la croissance de microorganismes, par exemple de bactéries quel que soit le mode de culture, et/ou de tester l'effet ou la susceptibilité (CMI) de composés/agents physiques ou chimiques, par exemple des antimicrobiens, et/ou de permettre la prise en compte des facteurs environnementaux et/ou de reproduire/mimer des conditions environnementales.

### Description de l'invention

La présente invention résout les inconvénients et obstacles de l'art antérieur via la fourniture d'un milieu de culture de microorganismes.

En particulier, la présente invention a pour objet un milieu de culture de microorganismes comprenant un hydrolysat enzymatique de protéines, un hydrolysat de protéines, un extrait de levure, du sérum de mammifère, du sang de mammifère, des débris cellulaires et au moins un sel, dans lequel le pH du milieu de culture est compris entre 7,5 et 8,5.

Les inventeurs ont démontré que la présente invention, en particulier le milieu selon l'invention permet avantageusement de cultiver des microorganismes et/ou de mesurer la croissance de microorganismes.

Les inventeurs ont également démontré de manière surprenante et inattendue que le milieu selon l'invention permet une coculture de différentes espèces de microorganismes et/ou de différents isolats au sein d'une même espèce. En outre, les inventeurs ont également démontré de manière surprenante que le milieu selon l'invention permet une coculture de différentes espèces de microorganismes et/ou de différents isolats au sein d'une même espèce pendant une longue durée, par exemple pendant au moins 6 semaines.

Les inventeurs ont également démontré que le milieu selon l'invention permet avantageusement de reproduire/ mimer des facteurs environnementaux et/ou des conditions environnementales dans lesquels les microorganismes se développent. En particulier, les inventeurs ont démontré de manière surprenante que le milieu selon l'invention permet avantageusement de reproduire les conditions environnementales notamment de plaies chez les mammifères. En outre, les inventeurs ont également démontré que le milieu selon l'invention permet avantageusement une coculture ou non de microorganismes, notamment dans différentes conditions, par exemple planctonique ou biofilm, et/ou de tester la croissance de microorganismes, par exemple de bactéries quel que soit le mode de culture, et/ou de tester l'effet ou la susceptibilité (CMI) de composés/agents physiques ou chimiques, par exemple des antimicrobiens, et/ou de permettre la prise en compte des facteurs environnementaux et/ou de reproduire/mimer des conditions environnementales.

Avantageusement, les inventeurs ont démontré que le milieu selon l'invention permet de simuler l'environnement d'une plaie, d'une part par les constituants, et d'autre part par le pH. En effet, le milieu selon l'invention a avantageusement un pH alcalin, par exemple entre 7,5 et 8,5, comprend du sérum, du sang, de préférence hémolysé, des débris cellulaires, notamment de kératinocytes, des sels minéraux. En outre, le milieu selon l'invention peut avantageusement comprendre du glucose et des protéines. Le milieu selon l'invention permet donc avantageusement de reproduire l'ensemble des caractéristiques et propriétés de plaies, par exemple tel que décrit par Cutting, 2003 [11].

Avantageusement, les inventeurs ont démontré que le milieu selon l'invention permet la coculture de plusieurs isolats au sein d'une même espèce, en particulier de bactéries. En outre, les inventeurs ont démontré que le milieu de culture selon l'invention permet avantageusement, par exemple une coculture de *Staphylococcus aureus* et *Pseudomonas aeruginosa,* sans être restreint à ces 2 espèces bactériennes, qui correspondent et représentent les bactéries les plus fréquemment isolées dans une plaie, avantageusement dans une plaie chronique.

Les inventeurs ont également démontré que le milieu selon l'invention permet notamment la coculture de bactéries à Gram positif et à Gram négatif.

Les inventeurs ont également démontré que le milieu selon l'invention permet avantageusement de tester l'efficacité de composés, notamment d'antimicrobiens existants, par exemple recommandés pour le traitement d'infections, mais également l'efficacité de nouvelles molécules quelles que soient les conditions de culture et/ou les microorganismes.

Dans la présente, le milieu de culture de microorganisme peut être un milieu aqueux.

Dans la présente par microorganismes, on entend tout microorganisme connu de l'homme du métier. Il peut s'agir par exemple d'une bactérie ou d'un champignon. Il peut s'agir également de cellules procaryotes, par exemple toute bactérie connue de l'homme du métier, par exemple les bactéries comprises dans le groupe, sans être limité à celui-ci, constitué de *Acetobacter aurantius, Actinobacillus actinomycetemcomitans, Agrobacterium tumefaciens, Azorhizobium caulinodans, Azotobacter vinelandii, Bacillus anthracis, Bacillus brevis, Bacillus cereus, Bacillus fusiformis, Bacillus licheniformis, Bacillus megaterium, Bacillus stearothermophilus, Bacillus subtilis, Bacteroides gingivalis, Bacteroides melaninogenicus, Bartonella henselae, Bartonella quintana, Bordetella bronchiseptica, Bordetella pertussis, Borrelia burgdorferi, Branhamella catarrhalis, Brucella abortus, Brucella melitensis, Brucella suis, Burkholderia mallei, Burkholderia pseudomallei Calymmatobacterium granulomatis, Campylobacter coli, Campylobacter jejuni, Campylobacter pylori, Clostridium botulinum, Clostridioides difficile, Clostridium perfringens, Clostridium tetani, Clostridium welchii, Corynebacterium diphtheriae, Corynebacterium fusiforme, Ehrlichia chaffeensis, Enterococcus avium, Enterococcus durans, Enterococcus faecalis, Enterococcus faecium, Enterococcus galllinarum, Enterococcus maloratus, Escherichia coli, Francisella tularensis, Fusobacterium nucleatum, Gardnerella vaginalis, Haemophilus ducreyi, Haemophilus influenzae, Haemophilus parainfluenzae, Haemophilus pertussis, Haemophilus vaginalis, Helicobacter pylori, Klebsiella pneumoniae, Klebsiella rhinoscleromatis-Klebsiella oxytoca, Lactobacillus acidophilus, Lactobacillus casei, Lactococcus lactis, Legionella pneumophila, Methanobacterium extroquens, Microbacterium multiforme, Micrococcus luteus, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycoplasma fermentans, Mycoplasma genitalium, Mycoplasma hominis, Mycoplasma pneumoniae Neisseria gonorrhoeae, Neisseria meningitidis, Nocardia asteroides Pasteurella multocida, Pasteurella tularensis, Porphyromonas gingivalis, Pseudomonas aeruginosa, Pseudomonas maltophilia, Rhizobium radiobacter, Rickettsia prowazekii, Rickettsia mooseri, Rickettsia psittaci, Rickettsia quintana, Rickettsia rickettsii, Rickettsia trachomae, Rochalimaea henselae, Rochalimaea quintana, Rothia dentocariosa, Salmonella enteritidis, Salmonella typhi, Salmonella typhimurium, Serratia marcescens, Shigella dysenteriae, Staphylococcus aureus, Staphylococcus epidermidis, Streptococcus agalactiae, Streptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis, Streptococcus sobrinus, Treponema pallidum, Vibrio cholerae, Vibrio comma, Vibrio parahemolyticus, Vibrio vulnificus,* , etc. Il peut s'agir par exemple de bactéries fréquemment isolées dans les plaies chroniques, par exemple *Staphylococcus aureus,* les staphylocoques à coagulase négative, *Pseudomonas aeruginosa,* par exemple des bactéries appartenant à la famille des *Enterobacteriaceae,* des bactéries appartenant au genre *Enterococcus,* des *streptocoques béta-hémolytiques (Streptococcus pyogenes)* ou des corynébactéries.

Il peut s'agir par exemple de tout champignon connu de l'homme du métier, par exemple de champignons pathogènes ou non pathogènes, par exemple de champignons responsables de pathologies, par exemple en santé humaine ou non, de champignons environnementaux, des champignons choisis dans le groupe comprenant les levures, par exemple *Candida* et/ou *Cryptococcus,* par exemple de levures responsables de pathologies, par exemple en santé humaine, par exemple de candidoses et/ou de cryptococcose, et/ou le groupe comprenant les moisissures, par exemple *Aspergillus,* par exemple de moisissures responsables de pathologie, par exemple *Cladosporium,* par exemple en santé humaine, par exemple d'aspergillose.

Selon l'invention, par hydrolysat enzymatique de protéines, on entend par exemple un hydrolysat trypsique de protéines et/ou un hydrolysat pepsique de protéines.

Selon l'invention, par hydrolysat trypsique de protéines on entend tout hydrolysat trypsique de protéines connu de l'homme du métier. Il peut s'agir d'un hydrolysat trypsique de protéines choisies dans le groupe comprenant de la peptone d'origine animale, de la peptone d'origine laitière, de la peptone d'origine végétale, de la peptone mycélienne et/ou un quelconque mélange de ceux-ci.

Selon l'invention, par hydrolysat pepsique de protéines on entend tout hydrolysat pepsique de protéines connu de l'homme du métier. Il peut s'agir d'un hydrolysat pepsique de protéines choisies dans le groupe comprenant de la peptone d'origine animale, de la peptone d'origine laitière, de la peptone d'origine végétale, de la peptone mycélienne et/ou un quelconque mélange de ceux-ci.

Selon l'invention, par peptone d'origine animale on entend toute peptone d'origine animale connue de l'homme du métier. Il peut s'agir par exemple de la peptone de mammifère. Il peut s'agir par exemple de peptone de fibres musculaires, par exemple de peptone de muscles striés squelettiques et/ou de cœur.

Selon l'invention, par peptone d'origine laitière on entend toute peptone d'origine laitière connue de l'homme du métier. Il peut s'agir par exemple de la peptone de caséine, de lactosérum.

Selon l'invention, par peptone d'origine végétale on entend toute peptone d'origine végétale connue de l'homme du métier. Il peut s'agir par exemple de la peptone de soja, de la peptone de coton, de maïs, de fève, de blé.

Selon l'invention, par peptone mycélienne on entend toute peptone mycélienne connue de l'homme du métier. Il peut s'agir par exemple de la peptone de levure.

Avantageusement, l'hydrolysat enzymatique de protéines est un l'hydrolysat pepsique de protéines. Avantageusement, l'hydrolysat pepsique de protéines est de la peptone d'origine animale, de préférence de la peptone de viande, de préférence de la peptone de fibres musculaires.

Selon l'invention, l'hydrolysat enzymatique de protéines, de préférence l'hydrolysat pepsique peut être dans une solution aqueuse.

Selon l'invention, la concentration d'hydrolysat enzymatique de protéines dans le milieu de culture peut être comprise de 0,75 à 0,85 % en poids par rapport au poids total du milieu de culture. Par exemple, de préférence, la concentration d'hydrolysat enzymatique de protéines dans le milieu de culture peut être égale à 0,795 % en poids par rapport au poids total du milieu de culture.

Avantageusement, lorsque l'hydrolysat enzymatique de protéines est un l'hydrolysat pepsique de protéines, la concentration d'hydrolysat pepsique de protéines dans le milieu de culture peut être comprise de 0,75 à 0,85 % en poids par rapport au poids total du milieu de culture. Par exemple, de préférence, la concentration d'hydrolysat pepsique de protéines dans le milieu de culture peut être égale à 0,795 % en poids par rapport au poids total du milieu de culture.

Selon l'invention, par hydrolysat de protéines, on entend tout hydrolysat de protéines adapté connue de l'homme du métier. Il peut s'agir par exemple d'un hydrolysat de protéines obtenu par hydrolyse enzymatique, par hydrolyse chimique, par exemple par hydrolyse acide ou hydrolyse alcaline. Il peut s'agir par exemple d'un hydrolysat de protéines d'origine animale, par exemple un hydrolysat de caséine(s), par exemple un hydrolysat de lactalbumine, de lactoglobuline, d'albumine. De préférence, l'hydrolysat de protéines peut être un hydrolysat de lactalbumine. De manière plus préférée, l'hydrolysat de protéines peut être un hydrolysat de lactalbumine.

Selon l'invention, l'hydrolysat de protéines peut être dans une solution aqueuse. Il peut s'agir par exemple d'un milieu Bolton.

Selon l'invention, la concentration d'hydrolysat de protéines dans le milieu de culture peut être comprise de 0,395 à 0,42 % en poids par rapport au poids total du milieu de culture. Par exemple, de préférence, la concentration d'hydrolysat de protéines dans le milieu de culture peut être égale à 0,398 % en poids par rapport au poids total du milieu de culture.

Selon l'invention, par extrait de levure on entend tout extrait de levure connu de l'homme du métier. Il peut s'agir par exemple d'un extrait de levure obtenu par lyse de levures par chauffage suivi d'une digestion enzymatique par les propres enzymes de la levure, et élimination des composants insolubles de la paroi par centrifugation et filtration. Il peut s'agir par exemple d'un extrait de levure disponible dans le commerce, par exemple commercialisé par la société Grosseron sous la référence commerciale Biokar (marque déposée). Il peut s'agir par exemple d'un extrait de levure commercialisé par la société Difco sous la marque Bacto (marque déposée) Dehydrated Culture Media, d'un extrait de levure commercialisé par la société Conda sous la référence 1702, par la société BD Bioscience sous la marque Bacto (marque déposée Yeast Extract).

Selon l'invention, l'extrait de levure peut être dans une solution aqueuse. Il peut s'agir par exemple d'un milieu Bolton.

Selon l'invention, la concentration de l'extrait de levure dans le milieu de culture peut être comprise de 0,395 à 0,42 % en poids par rapport au volume total du milieu de culture. Par exemple, de préférence, la concentration de l'extrait de levure dans le milieu de culture peut être égale à 0,398 % en poids par rapport au volume total du milieu de culture.

Selon l'invention le sérum de mammifère peut être du sérum humain ou animal. Il peut s'agir par exemple de sérum d'un animal d'élevage, d'un animal de compagnie, ou de tout autre animal. Par exemple, l'animal d'élevage peut être choisi dans le groupe comprenant les bovins, porcins, ovins, caprins, camelins, canins, équins, murins, primates. Par exemple, l'animal de compagnie peut être choisi dans le groupe comprenant les canins et les félins. Avantageusement, le sérum de mammifère est du sérum humain. Selon l'invention, le sérum de mammifère peut être un extrait de sérum de mammifère. Dans la présente par extrait de sérum, on entend tout ou partie du sérum. Il peut s'agir par exemple de sérum ayant subi un traitement et/ou procédé de préparation. Il peut s'agir, par exemple d'un sérum décomplémenté, d'un sérum stérilisé.

Dans la présente, un sérum décomplémenté peut être obtenu par tout procédé adapté connu de l'homme du métier. Il peut s'agir par exemple d'un procédé de décomplémentation comprenant une étape de chauffage du sérum à une température comprise de 50 à 60 °C, par exemple de 56 °C pendant un temps compris de 25 à 35 minutes, par exemple 30 minutes. Avantageusement, le procédé de décomplémentation permet de détruire les composants thermolabiles du complément présents dans le sérum.

Dans la présente le sérum stérilisé peut être obtenu par tout procédé de stérilisation adapté connu de l'homme du métier.

Avantageusement, le sérum de mammifère est du sérum de mammifère décomplémenté. Avantageusement, le sérum de mammifère est du sérum humain décomplémenté.

Avantageusement, lorsque le milieu de culture comprend du sérum de mammifère, en particulier du sérum humain, le milieu de culture peut permettre de reproduire/mimer des conditions environnementales, par exemple de plaies.

Selon l'invention, la concentration du sérum de mammifère ou d'extrait de sérum de mammifère dans le milieu de culture peut être comprise de 17 à 23 % en volume par rapport au volume total du milieu de culture. Par exemple, de préférence, la concentration du sérum de mammifère ou extrait de sérum de mammifère dans le milieu de culture peut être égale à 20% en volume par rapport au volume total du milieu de culture.

Selon l'invention, le sang de mammifère peut être tout sang de mammifère adapté connu de l'homme du métier. Il peut s'agir par exemple de sang de mammifère choisi dans le groupe comprenant du sang humain ou animal. Il peut s'agir par exemple de sang d'un animal d'élevage, d'un animal de compagnie, ou de tout autre animal. Par exemple, l'animal d'élevage peut être choisi dans le groupe comprenant les bovins, porcins, ovins, caprins, camelins, canins, équins, murins, primates. Par exemple, l'animal de compagnie peut être choisi dans le groupe comprenant les canins et les félins. Avantageusement, le sang de mammifère est du sang humain. Le sang humain peut être, par exemple du sang humain quel que soit le groupe et/ou rhésus. Il peut s'agir par exemple de sang humain avec un groupe sanguin choisi dans le groupe A+, A-, B+, B-, AB+, AB-, O+ et O- ou un quelconque mélange de ceux-ci. Il peut s'agir par exemple de sang humain de groupe sanguin AB+. Avantageusement, lorsque le sang humain est de groupe sanguin AB+, il présente l'ensemble des antigènes de surface.

Selon l'invention, le sang de mammifère peut être un extrait de sang de mammifère. Dans la présente par extrait de sang, on entend tout ou partie du sang. Il peut s'agir par exemple de sang ayant subi un traitement et/ou procédé de préparation. Il peut s'agir, par exemple de sang hémolysé, d'un surnageant de sang après préparation par un procédé comprenant une étape d'hémolyse, de préférence physique, puis décantation. Avantageusement, l'extrait de sang de mammifère, de préférence humain, est un surnageant de sang hémolysé.

Selon l'invention, la concentration de sang et/ou d'extrait de sang de mammifère est comprise de 0,5 à 1 % en volume par rapport au volume total du milieu de culture. Par exemple, de préférence, la concentration de sang et/ou d'extrait de sang de mammifère est égale à 0,5 % en volume par rapport au volume total du milieu de culture.

Selon l'invention, le sel peut être tout sel adapté connu de l'homme du métier. Il peut s'agir par exemple d'un sel choisi dans le groupe comprenant le NaCI, KCI, CuCl₂, ZnCl₂ ou un mélange de ceux-ci. Avantageusement, le sel est du NaCI. Selon l'invention, la concentration de sel dans le milieu de culture peut être comprise de 0,395 à 0,42 % en poids par rapport au volume total du milieu de culture. Par exemple, de préférence, la concentration de sel dans le milieu de culture peut être égale à 0,398 % en poids par rapport au volume total du milieu de culture.

Selon l'invention, le milieu de culture peut comprendre des débris cellulaires. Il peut s'agir par exemple de débris de toute cellule de mammifères connus de l'homme du métier. Il peut s'agir par exemple de débris cellulaires après la mort d'une cellule, de débris cellulaires en raison de dommages causés par des facteurs physiques, chimiques, infectieux, biologiques, nutritionnels ou immunologiques défavorables. Il peut s'agir par exemple de l'ensemble des déchets organiques laissés après la mort d'une cellule, par exemple tels que ceux décrits dans le document Ölander M et al. Image-Based Quantification of Cell Debris as a Measure of Apoptosis. Anal Chem. 2019 May 7;91(9):5548-5552. doi: 10.1021/acs.analchem.9b01243. Epub 2019 Apr 25. PMID: 31001971 [22].

Dans la présente, les débris cellulaires peuvent être obtenus par tout procédé adapté connu de l'homme du métier. Il peut s'agir par exemple d'un procédé décrit dans le document Smucker and Pfister 1975 Applied Microb., 30 : 445-449 (Liquid Nitrogen Cryo-Impacting: a New Concept for Cell Disruption [23]). Il peut s'agir par exemple d'un procédé comprenant une lyse cellulaire, par exemple comprenant au moins une étape de congélation des cellules et décongélation des cellules.

Selon l'invention, les débris cellulaires peuvent être d'une taille comprise de 0,5µm à 10µm.

Selon l'invention, les débris cellulaires peuvent être choisis parmi des débris de kératinocytes, de mélanocytes, de fibroblastes, d'histiocytes, de mastocytes ou de macrophages, ou un quelconque mélange de ceux-ci. Il peut s'agir par exemple de débris de lignées cellulaires, par exemple de lignées de cellules kératinocytaires, par exemple les cellules HaCaT, de lignées de cellules mélanocytaires, par exemple les cellules PIG1, de lignées de fibroblastes dermiques humains, par exemple les cellules HDFa, de lignées de cellules histiocytaires, par exemple les cellules U937, de lignées de cellulaires de mastocytes, par exemple de cellules HMC-1.1, de lignées de cellules monocytaires, par exemple de cellules THP-1. Selon l'invention, les cellules de lignées cellulaires peuvent être en outre différenciées, par exemple pour les lignées de cellules monocytaires, en macrophages, et/ou différenciées en cultures primaires de cellules et/ou en tissus.

Selon l'invention, la concentration de débris cellulaires et/ou de cellules sous forme de débris cellulaires dans le milieu de culture peut être comprise de 1x10⁸ à 1x10¹⁰ débris par litre de milieu de culture. Par exemple, de préférence, la concentration de débris cellulaires et/ou de cellules sous forme de débris cellulaires dans le milieu de culture peut être égale 1x10⁹ débris par litre de milieu de culture et/ou cellules sous forme de débris par litre de milieu de culture.

Selon l'invention, lorsque le milieu de culture comprend un mélange de débris cellulaire, par exemple des débris de kératinocytes, de mélanocytes et/ou de macrophages, le milieu peut comprendre un rapport de débris de kératinocytes sur les débris de mélanocytes et/ou macrophages compris de 1 à 99.

Selon l'invention, le pH du milieu de culture peut être neutre ou basique. Avantageusement, le pH du milieu de culture est basique. Le pH du milieu de culture peut être compris de 7,5 à 8,5. Avantageusement, le pH du milieu de culture peut être égal à 8.

Selon l'invention, le milieu de culture peut comprendre en outre un tampon. Il peut s'agir par exemple de tout tampon et/ou solution tampon adapté pour mélange avec un milieu de culture de microorganismes comprenant un hydrolysat enzymatique de protéines, un hydrolysat de protéines, un extrait de levure, du sérum de mammifère, du sang de mammifère, des débris cellulaires et au moins un sel, connu de l'homme du métier. Il peut s'agir par exemple d'un tampon choisi dans le groupe comprenant du tampon Hepes et du tampon bicarbonate. Il peut s'agir par exemple de tampon Hepes à une molarité comprise de 5 à 20 mM, par exemple égale à 10 mM. Il peut s'agir par exemple de tampon bicarbonate à une molarité comprise de 5 à 20 mM, par exemple égale à 10 mM.

Avantageusement, le tampon peut permettre d'ajuster le pH du milieu, par exemple entre 7,5 et 8,5. En outre, le tampon peut avantageusement permettre d'ajuster le pH du milieu de culture afin qu'il corresponde et/ou se rapproche du pH pour avantageusement reproduire/ mimer des facteurs environnementaux et/ou des conditions environnementales dans lesquels les microorganismes se développent.

Selon l'invention, le milieu de culture peut comprendre en outre au moins un composé choisi parmi l'acide alpha-cétoglutarique, le pyruvate de sodium, le métabisulfite de sodium, le carbonate de sodium, l'hémine, un sel ou un mélange de ceux-ci.

Avantageusement, le milieu de culture peut comprendre en outre au moins deux, au moins trois, au moins quatre composés choisis parmi l'acide alpha-cétoglutarique, le pyruvate de sodium, le métabisulfite de sodium, le carbonate de sodium, l'hémine.

Avantageusement, le milieu de culture peut comprendre en outre de l'acide alpha-cétoglutarique, du pyruvate de sodium, du métabisulfite de sodium, du carbonate de sodium et de l'hémine

Selon l'invention, la concentration d'acide alpha-cétoglutarique dans le milieu de culture peut être comprise de 0,0792 à 0,0798 % en poids par rapport au volume total du milieu de culture, de préférence égale à 0,0795 % en poids par rapport au volume total du milieu de culture.

Selon l'invention, la concentration de pyruvate de sodium dans le milieu de culture peut être comprise de 0,0394 à 0,04 % en poids par rapport au volume total du milieu de culture, de préférence égale à 0,0397 % en poids par rapport au volume total du milieu de culture.

Selon l'invention, la concentration de métabisulfite de sodium dans le milieu de culture peut être comprise de 0,0394 à 0,04 % en poids par rapport au volume total du milieu de culture, de préférence égale à 0,0397 % en poids par rapport au volume total du milieu de culture.

Selon l'invention, la concentration d'hémine dans le milieu de culture peut être comprise de 0,00792 à 0,00798 % en poids par rapport au volume total du milieu de culture, de préférence égale à 0,00795 % en poids par rapport au volume total du milieu de culture.

Selon l'invention, la concentration de carbonate de sodium dans le milieu de culture peut être comprise de 0,0474 à 0,0480 % en poids par rapport au volume total du milieu de culture, de préférence égale à 0,0477 % en poids par rapport au volume total du milieu de culture.

Selon l'invention, l'acide alpha-cétoglutarique et/ou le pyruvate de sodium et/ou le métabisulfite de sodium et/ou le carbonate de sodium et/ou l'hémine peut être dans une solution aqueuse. Il peut s'agir par exemple d'un milieu Bolton.

Selon l'invention, l'hydrolysat enzymatique de protéines et/ou l'hydrolysat de protéines et/ou l'extrait de levure et/ou le sel peut être dans une solution aqueuse. Il peut s'agir par exemple d'un milieu Bolton.

Selon l'invention, le milieu de culture peut comprendre les ingrédients tels que défini dans le Tableau 1 ci-dessous :

**Tableau 1 : exemple de composition de composants du milieu de culture**

| **Composants** |
|---|
| Peptone de viande |
| Hydrolysat de lactalbumine |
| Extrait de levure |
| NaCl |
| Acide alpha-cétoglutarique |
| Pyruvate de sodium |
| Métabisulfite de sodium |
| Carbonate de sodium |
| Hémine |
| Sang humain hémolysé |
| Sérum humain décomplémenté |
| Débris cellulaire de kératinocytes |
| HEPES ou bicarbonate de sodium 10mM final, ajusté à pH 8,0 avec NaOH |

Selon l'invention, le milieu de culture peut comprendre les ingrédients tels que défini dans le Tableau 2 ci-dessous :

**Tableau 2 : exemple de composition du milieu de culture**

| **Ingrédients pour 1L de milieu** | **Quantité** | **Concentration** (Pourcentage en poids par rapport au poids total de la composition) |
|---|---|---|
| Peptone de viande | 7,95 g | 0,795 % |
| Hydrolysat de lactalbumine | 3,98 g | 0,398 % |
| Extrait de levure | 3,98 g | 0,398 % |
| NaCl | 3,98 g | 0,398 % |
| Acide alpha-cétoglutarique | 0,79 g | 0,0795 % |
| Pyruvate de sodium | 0,39 g | 0,0397 % |
| Métabisulfite de sodium | 0,39 g | 0,0397 % |
| Carbonate de sodium | 0,48 g | 0,048 % |
| Hémine | 8 mg | 0,00795 % |
| Sang humain hémolysé | 500 µl | 0,5 % |
| Sérum humain décomplémenté | 200 ml | 20% |
| Débris cellulaire de kératinocytes | 1x10⁹ débris | |
| HEPES ou bicarbonate de sodium 100mM, ajusté à pH 8,0 avec NaOH | 100 ml | |
| Eau déminéralisée ultrapure | QSP | |

Selon l'invention, le milieu de culture peut comprendre un ou plusieurs adjuvants connus de l'homme du métier adapté au milieu de culture de microorganismes. Il peut s'agir par exemple d'un ou plusieurs adjuvant(s) choisi(s) parmi des agents, des vitamines et les provitamines, des protéines, des agents séquestrants, des agents alcanisants, des agents acidifiants, des agents réducteurs, des agents anti-oxydants, des colorants, une source de carbone, par exemple du glucose, du mannose, du fructose.

Selon l'invention, le milieu de culture peut être avantageusement associé à un milieu de culture sélectif, un milieu de culture enrichi, un milieu différentiel, un milieu chromogène.

Dans la présente, le milieu de culture sélectif peut être tout milieu sélectif adapté connu de l'homme du métier. Il peut s'agir par exemple d'un milieu liquide Chapman, également désigné Mannitol salt (ou Chapman), par exemple d'un milieu solide Chapman, également désigné Mannitol salt (ou Chapman) comprenant de l'agar.

Dans la présente le milieu de culture enrichi peut comprendre du glucose et/ou au moins un antimicrobien.

L'antimicrobien peut être par exemple au moins un antibiotique et/ou au moins un antiseptique et/ou un antifongique.

L'antibiotique dans le milieu de culture enrichi peut être tout antibiotique connu de l'homme du métier adapté. Il peut s'agir par exemple d'un antibiotique qui peut être choisi en fonction de la résistance naturelle et/ou acquise du microorganisme à cultiver et/ou de la souche du microorganisme considérée et/ou d'une résistance apportée, par exemple par un plasmide, a un microorganisme. Il peut s'agir par exemple d'un antibiotique choisi dans le groupe comprenant l'acide fusidique, les aminosides, les bêta-lactamines (les pénicillines, associés ou non à des inhibiteurs de bêta-lactamases, les céphalosporines, les carbapénèmes, les monobactames), la cyclosérine, la daptomycine, la fosfomycine, les lincosamides, les macrolides et kétolides, la mupirocine, les imidazolés, les nitrofuranes, les oligosaccharides, les oxazolidinones, les polypeptides: bacitracine, glycopeptides, lipoglycopeptides, polymyxines en particulier la colistine, les quinolones, les rifamycines, les sulfamides et les diaminopyridines, les synergistines, les cyclines et glycylcyclines, les phénicolés, le cotrimoxazole.

L'antiseptique peut être choisi par exemple parmi la povidone iodée, le Dakin (hypochlorite de sodium à 0,5% additionné de permanganate de potassium), le PHMB (PolyHexaMethylène de Biguanide) ou l'octenidine. Par exemple le milieu de culture enrichi peut comprendre une concentration de glucose supérieure à 0,4 g/L, par exemple une concentration de glucose de 0,5 g/L de milieu de culture.

Il peut s'agir par exemple d'un antifongique choisi dans le groupe comprenant le Miconazole, le Ketoconazole, le Clotrimazole, l'Éconazole, le Bifonazole, le Butoconazole, le Fenticonazole, l'Isoconazole, l'Oxiconazole, le Sertaconazole, le Sulconazole, le Thiabendazole, le Tioconazole, le Fluconazole, l'Itraconazole, l'Isavuconazole, le Ravuconazole, le Posaconazole, le Voriconazole, la Terbinafine, l'Amorolfine, la Naftifine, la Butenafine, la Mycosubtiline ou un quelconque mélange de ceux-ci.

Avantageusement, lorsque le milieu comprend une concentration de glucose supérieure à 0,4g/L, par exemple une concentration de glucose de 0,5g/L de milieu de culture, elle peut correspondre avantageusement à la concentration de glucose présente dans les plaies de patients diabétiques, en particulier dans des exsudats de plaies de patients diabétiques.

Dans la présente, le milieu de culture sélectif peut être tout milieu sélectif connu de l'homme du métier. Il peut s'agir par exemple d'un milieu de culture sélectif comprenant du NaCI et/ou du bleu de méthylène et/ou de l'éosine jaune et/ou au moins un antibiotique et/ou au moins un fongicide et/ou au moins un antiseptique.

Avantageusement, lorsque le milieu sélectif comprend du chlorure de sodium (NaCI), par exemple à un taux de 5 à 15% (poids/volume), soit une concentration de 85 à 256 mM, il peut permettre de sélectionner des microorganismes halophiles.

Avantageusement, lorsque le milieu sélectif comprend du bleu de méthylène et/ou de l'éosine jaune, il peut avantageusement permettre une inhibition de la croissance des bactéries de type entérocoques et favoriser la croissance de microorganismes halophiles.

L'homme du métier, de part ces connaissances générales saura adapter la composition du milieu sélectif.

Dans la présente, le milieu de culture chromogène peut être tout milieu de culture chromogène adapté connu de l'homme du métier. Il peut s'agir par exemple d'un milieu de culture chromogène disponible dans le commerce. Il peut s'agir par exemple d'un milieu chromogène choisi dans le groupe comprenant le milieu UTI commercialisé par la société ThermoFisher, le milieu Rapid Enterococci ChromoSelect Agar (marque déposée) commercialisé par la société Sigma Aldrich, le milieu Candiselect (marque déposée) commercialisé par la société Bio-Rad, le milieu chromID (marque déposée) *S*. *aureus* commercialisé par la société bioMérieux,̅ le milieu MRSA *Select* (marque déposée) commercialisé par la société Bio-Rad.

Selon l'invention, le milieu de culture peut comprendre en outre un gélifiant. Par exemple le milieu de culture peut comprendre un gélifiant choisi dans le groupe comprenant l'agar-agar, un carraghénane et/ou un quelconque mélange de ceux-ci. De préférence, le gélifiant peut être de l'agar-agar. Selon l'invention, la concentration de gélifiant dans le milieu de culture peut être comprise de 0,8 à 2,0% en poids/volume du milieu de culture. Par exemple, de préférence, la concentration de gélifiant dans le milieu de culture peut être de 1% en poids/volume de milieu de culture. Avantageusement, lorsque le milieu comprend un gélifiant, le milieu selon l'invention peut être avantageusement sous forme solide.

Les inventeurs ont également démontré de manière surprenante et inattendue que le milieu selon l'invention permet de cultiver tout type de microorganismes quel que soit son mode de croissance.

Aussi, la présente invention a également pour objet l'utilisation du milieu selon l'invention dans un procédé de culture de microorganismes. Il peut s'agir par exemple de tout procédé de culture de microorganismes adapté connu de l'homme du métier. Il peut s'agir par exemple d'un procédé aérobie de culture de microorganismes ou un procédé anaérobie de culture de microorganismes ou un procédé de culture de microorganismes microaérophile. Il peut s'agir par exemple de tout procédé adapté de culture de microorganismes aérobie connu de l'homme du métier. Il peut s'agir par exemple d'une culture en milieu liquide. Il peut s'agir par exemple d'une culture en milieu gélosé, par exemple sur boite de Pétri. Il peut s'agir par exemple d'incuber les milieux dans une étuve à une température optimale de croissance, par exemple 37°C. Il peut s'agir par exemple d'incuber les milieux pendant un temps permettant la croissance du microorganisme, par exemple 24h. Il peut s'agir à titre d'exemple du procédé décrit dans le document Denis, F., Cattoir, V., Martin, C., Ploy, M. C., & Poyart, C. (2016). Bactériologie médicale: techniques usuelles. Elsevier Masson. Il peut s'agir plus généralement de tout procédé adapté de culture de microorganismes anaérobie connu de l'homme du métier. Il peut s'agir par exemple d'un procédé anaérobie de culture de microorganismes, par exemple dans lequel le milieu est dans un contenant en atmosphère anaérobie. Il peut s'agir par exemple d'un procédé anaérobie de culture de microorganismes, par exemple dans lequel le contenant de culture comprenant le milieu est dans une chambre anaérobie. Il peut s'agir par exemple d'un procédé anaérobie de culture de microorganismes, par exemple dans lequel le contenant de culture est un contenant adapté pour une culture de microorganismes en anaérobie. Il peut s'agir par exemple de tout contenant pour une culture de microorganismes en anaérobie connu de l'homme du métier, il peut s'agir par exemple d'un contenant clos. Il peut s'agir par exemple d'un sachet d'anaérobiose, par exemple un sachet d'anaérobiose commercialisé par la société fisher scientific, par exemple commercialisé sous la référence commerciale AnaeroGen (marque déposée) Compact Oxoido, par exemple un sachet d'anaérobiose commercialisé sous la référence commerciale AN0010, AnaeroGen Compact Oxoid [24]. Il peut s'agir par exemple de tout procédé adapté de culture de microorganismes microaérophile connu de l'homme du métier. Il peut s'agir par exemple de milieux faiblement gélosés présentant des gradients de teneur en oxygène [25].

Les inventeurs ont également démontré de manière surprenante et inattendue que le milieu selon l'invention permet de déterminer et/ou étudier la cinétique de croissance de microorganismes.

Aussi, la présente invention a également pour objet l'utilisation du milieu selon l'invention dans un procédé de détermination de la croissance de microorganismes. Il peut s'agir par exemple de tout procédé de détermination de la croissance de microorganismes, adapté, connu de l'homme du métier. Il peut s'agir par exemple d'un procédé de détermination de la croissance de microorganismes comprenant une mesure de la densité optique du milieu à 600nm, un procédé de détermination de la croissance de microorganismes comprenant une étape de dénombrement sur milieu gélosé de dilutions de prélèvements réalisés selon une cinétique, un procédé de détermination de la croissance de microorganismes comprenant une étape de mesure de la conductance du milieu, par exemple tel que décrit dans Richards et al., J. Phys. E, 1978, 11 : 560-568 [26], un procédé de détermination de la croissance de microorganismes comprenant une étape de mesure de l'ATP en luminescence.

Les inventeurs ont également démontré de manière surprenante et inattendue que le milieu selon l'invention permet de cultiver une pluralité de microorganismes quel que soit son mode de croissance.

La présente invention a également pour objet l'utilisation du milieu selon l'invention dans un procédé de culture d'une pluralité de microorganismes.

Dans la présente par pluralité de microorganisme on entend au moins deux types de microorganismes différents. Il peut s'agir par exemple d'au moins deux bactéries différentes, ou d'un mélange d'au moins deux champignons différents, ou d'un mélange d'au moins une bactérie et d'au moins un champignon.

Selon l'invention, la bactérie peut être telle que mentionnée ci-dessus.

Selon l'invention, le champignon peut être tel que mentionné ci-dessus.

Dans la présente, par mélange d'au moins deux bactéries différentes, on entend par exemple au moins deux bactéries d'espèces différentes et/ou de genre différent et/ou deux souches différentes.

Dans la présente, par mélange d'au moins deux champignons différents, on entend par exemple au moins deux champignons d'espèces différentes et/ou de genres différents et/ou deux souches différentes.

Il peut s'agir par exemple d'un mélange de différentes souches de bactéries, de champignons. Il peut s'agir par exemple d'un mélange de champignons du genre *Candida, Aspergillus, Cladosporium.* Il peut s'agir, par exemple, d'un mélange de champignons avec différentes souches d'*Aspergillus cibarius.* Il peut s'agir par exemple d'un mélange de champignons du genre *Candida.* Il peut s'agir par exemple d'un mélange de champignons comprenant des souches de *Candida albicans, Candida parapsilosis* ou *Candida auris.*

La présente invention a également pour objet l'utilisation du milieu selon l'invention dans un procédé de détermination de la cinétique de croissance des microorganismes. Il peut s'agir par exemple de tout procédé de détermination de la cinétique de croissance de microorganismes, adapté, connu de l'homme du métier. Il peut s'agir par exemple d'un procédé de détermination de la cinétique de croissance de microorganismes, par exemple tel que décrit dans Microbial growth kinetics (Panikov, 1995) [27].

Les inventeurs ont également démontré de manière surprenante et inattendue que le milieu selon l'invention peut être utilisé dans un procédé de détermination de la sensibilité de microorganismes à des composés, par exemple ayant un potentiel anti-microbien.

La présente invention a également pour objet l'utilisation du milieu selon l'invention dans un procédé de détermination de la sensibilité de microorganismes à un composé test.

Dans la présente par sensibilité de microorganismes à des composés on entend tout élément connu de l'homme du métier susceptible de modifier la croissance/développement de microorganismes et/ou capable de tuer un microorganisme.

Dans la présente par composé test on entend tout composé, par exemple naturel, obtenu par synthèse, hémi-synthèse chimique, ou obtenu par extraction, connu de l'homme du métier susceptible de modifier la croissance/développement de microorganismes et/ou capable de tuer un microorganisme. Il peut s'agir par exemple d'un biocide, d'un désinfectant, d'un produit de protection, d'un produit de lutte contre les espèces dites nuisibles et autre et/ou de tout biocide inclus dans la liste selon la directive 98/8/CE ou le règlement (UE) n°528/2012, d'un antibiotique, d'un antifongique.

Il peut s'agir par exemple d'un antibiotique choisi dans le groupe comprenant l'acide fusidique, les aminosides, les bêta-lactamines (les pénicillines, associés ou non à des inhibiteurs de bêta-lactamases, les céphalosporines, les carbapénèmes, les monobactames), la cyclosérine, la daptomycine, la fosfomycine, les lincosamides, les macrolides et kétolides, la mupirocine, les imidazolés, les nitrofuranes, les oligosaccharides, les oxazolidinones, les polypeptides: bacitracine, glycopeptides, lipoglycopeptides, polymyxines en particulier la colistine, les quinolones, les rifamycines, les sulfamides et les diaminopyridines, les synergistines, les cyclines et glycylcyclines, les phénicolés, le cotrimoxazole, les antifongiques, ou un quelconque mélange de ceux-ci.

Il peut s'agir par exemple d'un anti-fongique choisi dans le groupe comprenant le Miconazole, le Ketoconazole, le Clotrimazole, l'Éconazole, le Bifonazole, le Butoconazole, le Fenticonazole, l'Isoconazole, l'Oxiconazole, le Sertaconazole, le Sulconazole, le Thiabendazole, le Tioconazole, le Fluconazole, l'Itraconazole, l'Isavuconazole, le Ravuconazole, le Posaconazole, le Voriconazole, la Terbinafine, l'Amorolfine, la Naftifine, la Butenafine, la Mycosubtiline ou un quelconque mélange de ceux-ci.

Il peut s'agir également de tout nouveau composé, par exemple dont on cherche à mettre en évidence une éventuelle activité antimicrobienne, par exemple antiseptique, antibiotique et/ou antifongique, seule ou en association avec un ou plusieurs autres composés, ou d'un composé par exemple dont on cherche à mettre en évidence une éventuelle activité sur la formation de biofilm, ou sur un biofilm formé.

Selon l'invention, le composé test, par exemple un composé antimicrobien, antibiotique et/ou antifongique, peut-être en solution dans la solution, par exemple dans le milieu de culture, ou sous forme déshydratée ou lyophilisée, et/ou fixé sur la surface immergée dans ledit milieu.

Les moyens de fixation utilisables de l'antibiotique et/ou de l'antifongique peuvent être tout moyen connu de l'homme du métier pour la fixation d'un antibiotique et/ou d'un antifongique sur une surface. Il peut s'agir par exemple de moyens ou dispositifs disponibles dans le commerce, par exemple des supports de type microplaque, par exemple de moyens ou dispositifs commercialisés à titre d'exemple non limitatif, par les sociétés Nunc (Danemark), Corning (Etats-Unis), et Greiner Bio-One (Autriche).

Selon l'invention, on peut introduire dans le milieu de culture un ou plusieurs composés, par exemple un ou plusieurs antibiotiques.

Dans la présente, Il peut s'agir par exemple de tout procédé de détermination de la sensibilité de microorganismes à des composés, adapté, connu de l'homme du métier. Il peut s'agir par exemple d'un procédé de détermination de la sensibilité de microorganismes à des composés, par exemple tel que décrit dans le manuel édité par le Comité de l'antibiogramme de la Société Française de Microbiologie (CA-SFM ; www.sfm-microbiologie.org). Il peut s'agir par exemple d'un procédé de détermination de la concentration minimale inhibitrice (CMI) de microorganismes à des composés, par exemple un procédé comprenant les étape de préparation d'une gamme d'antibiotique de concentrations décroissantes par dilutions successives de raison 1/2 en milieu de culture liquide dans des tubes, de préparation d'un inoculum à 10⁵ bactéries par mL, de mise en contact de l'inoculum avec la gamme d'antibiotique, par exemple pendant 24h, par exemple à 37°C. La CMI correspond à la concentration en antibiotique du premier tube ne présentant ni trouble ni culot bactérien. Les étapes principales de détermination d'une CMI et procédé sont celles connues de l'homme du métier, par exemple celles décrites dans le Manuel édité par le Comité de l'antibiogramme de la Société Française de Microbiologie (CA-SFM ; www.sfm-microbiologie.org).

Dans la présente, les composés sont tels que définis ci-dessus.

La présente invention a pour objet un procédé de fabrication d'un milieu de culture selon l'invention comprenant les étapes suivantes :
a. hémolyse de sang de mammifère et décantage dudit sang hémolysé,
b. décomplémentation du sérum de mammifère et stérilisation du sérum décomplémenté,
c. mélange et stérilisation par chauffage ou filtration d'une solution aqueuse comprenant un hydrolysat enzymatique de protéines, de préférence un hydrolysat pepsique de protéines, d'un hydrolysat de protéines et d'un extrait de levure,
d. refroidissement du mélange stérile obtenu à l'étape c,
e. mélange dudit sang hémolysé décanté obtenu à l'étape a, dudit sérum décomplémenté obtenu à l'étape b, du mélange stérile refroidit obtenu à l'étape d, et de débris cellulaires, et obtention du milieu de culture.

Selon l'invention, le sang de mammifère peut être tel que mentionné ci-dessus.

Selon l'invention, le sérum de mammifère peut être tel que mentionné ci-dessus.

Selon l'invention, l'hydrolysat enzymatique de protéines peut être tel que mentionné ci-dessus.

Selon l'invention l'hydrolysat de protéines peut être tel que mentionné ci-dessus.

Selon l'invention, l'extrait de levure peut être tel que mentionné ci-dessus.

Selon l'invention, les débris cellulaires peuvent être tel que mentionné ci-dessus.

Selon l'invention, l'hémolyse de sang peut être réalisée par tout procédé, adapté, connu de l'homme du métier. Il peut s'agir par exemple d'un procédé d'hémolyse par congélation, d'un procédé d'hémolyse dans un milieu hypotonique.

Avantageusement, l'hémolyse du sang de mammifère peut être effectuée par congélation dudit sang de mammifère à une température comprise de -15°C à -25°C, de préférence à -20°C. Il peut s'agir par exemple d'une hémolyse du sang comprenant au moins un cycle de congélation - décongélation du sang. Par exemple le procédé d'hémolyse peut comprendre de 1 à 10 cycles de congélation, de préférence 4 cycles de congélation. La décongélation peut être réalisée à une température comprise de 15 à 25 °C, par exemple de 20°C.

Selon l'invention la durée de chaque cycle de congélation peut être compris entre 30 minutes et 2 heures.

Selon l'invention, le décantage du sang hémolysé peut être réalisé par tout procédé, adapté, connu de l'homme du métier. Il peut s'agir par exemple de décantage pendant un temps compris entre 1 heure et 2 heures.

Selon l'invention, la décomplémentation du sérum peut être réalisée par tout procédé adapté connu de l'homme du métier. Il peut s'agir par exemple d'un procédé de décomplémentation par chauffage, par exemple à une température comprise de 50°C à 60°C, de préférence à 56°C. Selon l'invention la durée du chauffage peut être comprise de 25 à 35 min, de préférence de 30 minutes.

Selon l'invention, la stérilisation du sérum décomplémenté peut être réalisée par tout procédé adapté connu de l'homme du métier. Il peut s'agir par exemple d'un procédé de stérilisation par filtration, par exemple, par filtration avec des filtres avec un diamètre de pores compris de 0,20 à 0,25 µm, de préférence de 0,22 µm.

Selon l'invention, le mélange d'un hydrolysat enzymatique de protéines, d'un hydrolysat de protéines et d'un extrait de levure dans une solution aqueuse peut être réalisé par tout procédé adapté connu de l'homme du métier. Il peut s'agir par exemple d'un procédé comprenant une étape d'agitation dans une solution aqueuse.

Selon l'invention, la stérilisation du mélange aqueux d'un hydrolysat enzymatique de protéines, d'un hydrolysat de protéines et d'un extrait de levure peut être réalisée par tout procédé adapté connu de l'homme du métier. Il peut s'agir par exemple d'un procédé de stérilisation par filtration, par exemple, par filtration des filtres avec un diamètre de pores compris de 0,20 à 0,25 µm, de préférence de 0,22 µm. Il peut s'agir par exemple d'un procédé de stérilisation par chauffage, par exemple à une température comprise de 100°C à 140°C, de préférence à 120°C pendant une durée comprise de 10 à 30 min, de préférence de 20 minutes.

Selon l'invention, le refroidissement du mélange stérilisé d'un hydrolysat enzymatique de protéines, d'un hydrolysat de protéines et d'un extrait de levure peut être réalisé par tout procédé de refroidissement adapté connu de l'homme du métier. Il peut s'agir par exemple d'un refroidissement à une température comprise de 20 à 37°C, par exemple à une température de 25°C.

Selon l'invention, l'étape e) de mélange peut être réalisée par tout procédé adapté connu de l'homme du métier. Selon l'invention l'étape de mélange de sang hémolysé décanté, de sérum décomplémenté, d'un mélange d'un hydrolysat enzymatique de protéines, d'un hydrolysat de protéines et d'un extrait de levure, et de débris cellulaires peut être réalisée par tout procédé adapté connu de l'homme du métier. Il peut s'agir par exemple d'un procédé comprenant une étape d'agitation, par exemple avec un agitateur magnétique.

Selon l'invention, le procédé de fabrication d'un milieu de culture peut comprendre en outre, préalablement à l'étape e) de mélange, une étape de lyse de cellules et obtention de débris cellulaires. Selon l'invention le procédé de lyse de cellules peut être réalisé par tout procédé, adapté, connu de l'homme du métier. Il peut s'agir par exemple d'un procédé décrit dans le document Smucker and Pfister 1975 Applied Microb., 30 : 445-449 (Liquid Nitrogen Cryo-Impacting: a New Concept for Cell Disruption) [23]. Il peut s'agir par exemple d'un procédé comprenant une lyse cellulaire, par exemple comprenant au moins une étape de congélation des cellules et décongélation des cellules.

Selon l'invention, les débris cellulaires peuvent être d'une taille comprise de 0,5µm à 10µm.

Selon l'invention, les cellules peuvent être toutes cellules telles que mentionnées ci-dessus.

Il peut s'agir par exemple de débris de lignées cellulaires, par exemple de lignées de cellules kératinocytaires, par exemple les cellules HaCaT, de lignées de cellules mélanocytaires, par exemple les cellules PIG1, de lignées de fibroblastes dermiques humains, par exemple les cellules HDFa, de lignées de cellules histiocytaires, par exemple les cellules U937, de lignées de cellulaires de mastocytes, par exemple de cellules HMC-1.1, de lignées de cellules monocytaires, par exemple de cellules THP-1. Selon l'invention, les cellules de lignées cellulaires peuvent être en outre différenciées, par exemple pour les lignées de cellules monocytaires, en macrophages, et/ou différenciées en cultures primaires de cellules et/ou en tissus.

Selon l'invention, le procédé de fabrication d'un milieu de culture peut comprendre en outre lors de l'étape c) l'ajout et/ou mélange avec un gélifiant. Selon l'invention, le gélifiant peut être tel que mentionné ci-dessus.

Selon l'invention, lorsque l'étape c) comprend en outre un gélifiant, la stérilisation du mélange peut être effectuée par chauffage tel que décrit ci-dessus.

D'autres avantages pourront encore apparaître à l'homme du métier à la lecture des exemples ci-dessous, illustrés par les figures annexées, donnés à titre illustratif.

### Brève description des figures

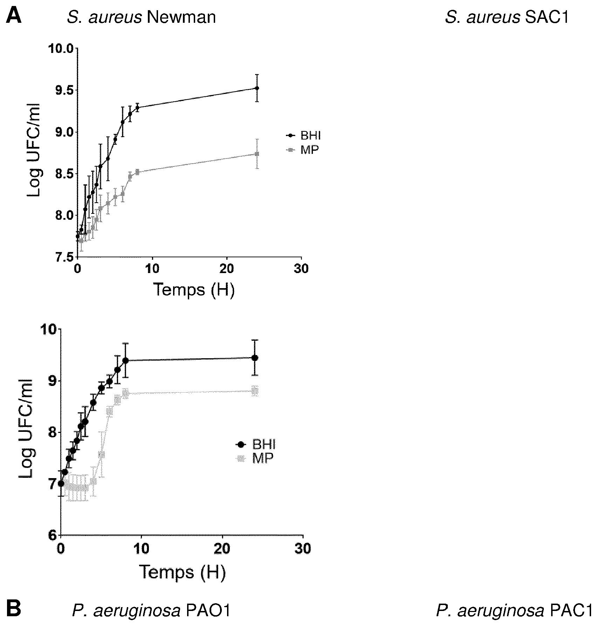

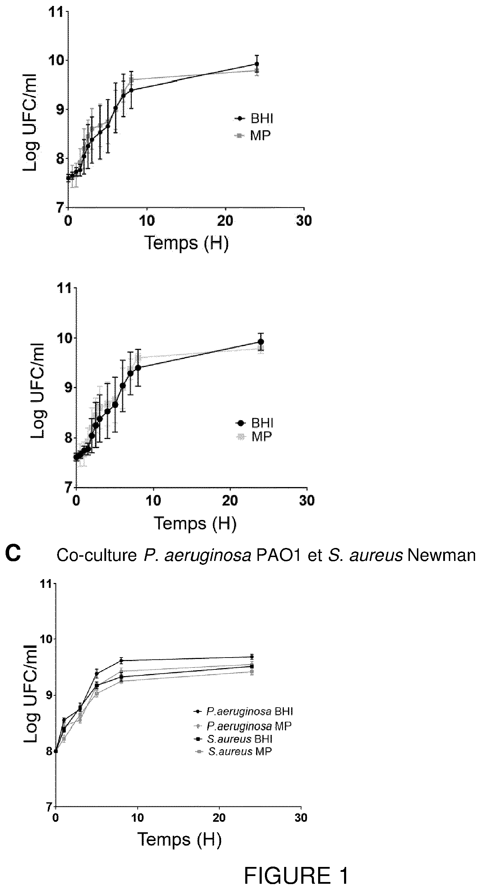

- La Figure 1 représente des courbes de croissance de bactéries en fonction du milieu de culture. Sur cette figure, les ordonnées correspondent au logarithme d'unités formant colonie par millilitre de milieu de culture (Log UFC/ml) et les abscisses, au temps en heure (H). La Figure 1A représente les courbes correspondant à la croissance de 2 souches de *S. aureus,* Newman (gauche) et SAC1 (droite), dans le milieu BHI (courbe avec indicateurs ronds) et un exemple de milieu selon l'invention (MP) (courbe avec indicateurs carrés). La Figure 1B représente les courbes correspondant à la croissance de 2 souches de *P. aeruginosa,* PAO1 (gauche) et PAC1 (droite), dans le milieu BHI (courbe avec indicateurs ronds) et un exemple de milieu selon l'invention (MP) (courbe avec indicateurs carrés). La Figure 1C représente les courbes correspondant à la croissance de 2 souches cliniques *S. aureus* SAC1 et *P. aeruginosa* PAC1, en coculture dans le milieu BHI (*P. aeruginosa* BHI ou *S. aureus* BHI) et un exemple de milieu selon l'invention (MP) (*P. aeruginosa* MP ou *S. aureus* MP). La mesure de différence significative, réalisée par le test de Student, entre les valeurs d'UFC/mL est exprimée, soit par (***) qui correspond à une p-value <0,001, soit par (**) qui correspond à une p-value <0,01.

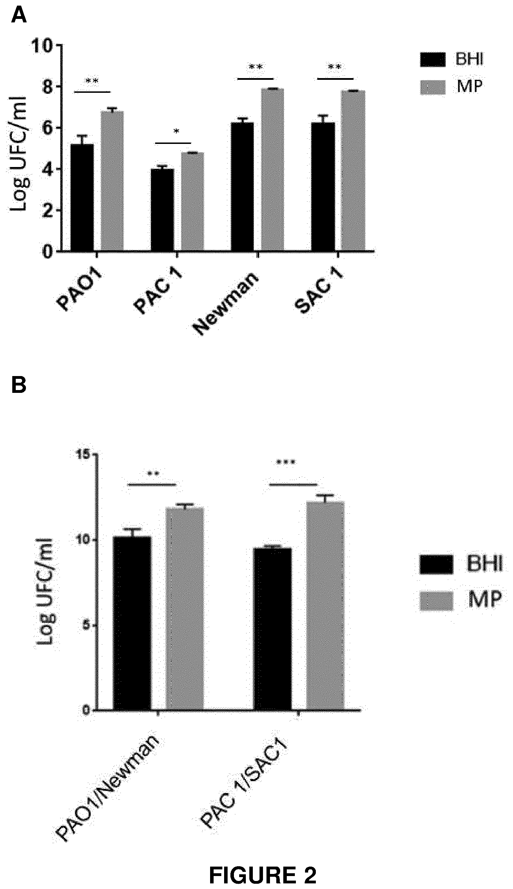

- La Figure 2 représente les résultats obtenus d'une détermination de l'adhésion et de la formation de biofilm par dénombrement à 24h. Sur cette figure, les ordonnées correspondent au logarithme d'unités formant colonie par millilitre de milieu de culture (Log UFC/ml). La Figure 2A représente un histogramme ou diagramme en bâton du nombre de bactéries dénombrées suite à des cultures séparées de souche *S. aureus* Newman (Newman), *S. aureus* SAC1 (SAC 1), *P. aeruginosa* PAO1 (PAO1) ou *P. aeruginosa* PAC1 (PAC1), pendant 24H dans un milieu BHI (BHI) (bâtons noirs) ou dans un exemple de milieu selon l'invention (MP) (bâtons gris).
   La Figure 2B montre sous forme d'histogramme le nombre de bactéries dénombrées suite à des co-cultures des souches de référence *S. aureus* Newman et *P. aeruginosa* PAO1 (PAO1/Newman), ainsi que du couple de souches cliniques *S. aureus* SAC1 et *P. aeruginosa* PAC1 (PAC 1/SAC1), pendant 24H dans un milieu BHI (BHI) (bâtons noirs) ou dans un exemple de milieu selon l'invention (MP) (bâtons gris).
   La mesure de différence significative, réalisée par le test de Student, entre les valeurs d'UFC est exprimée, soit par (***) qui correspond à une p-value <0,001, soit par (**) qui correspond à une p-value <0,01.

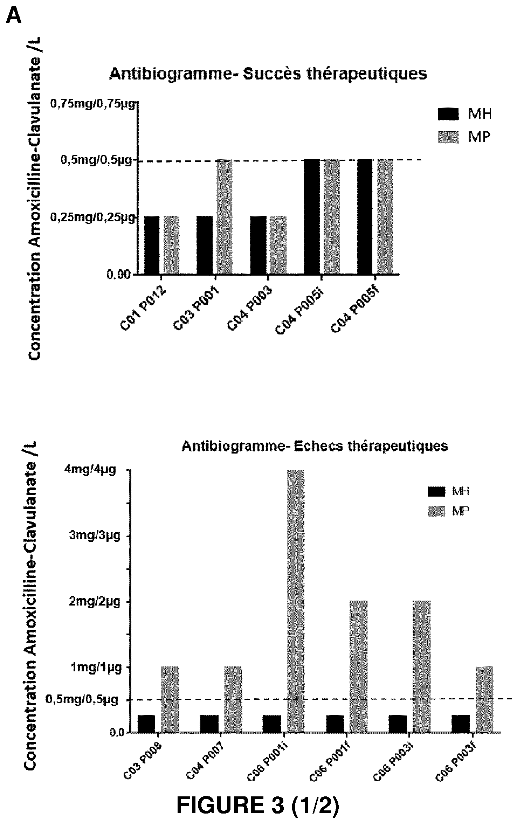

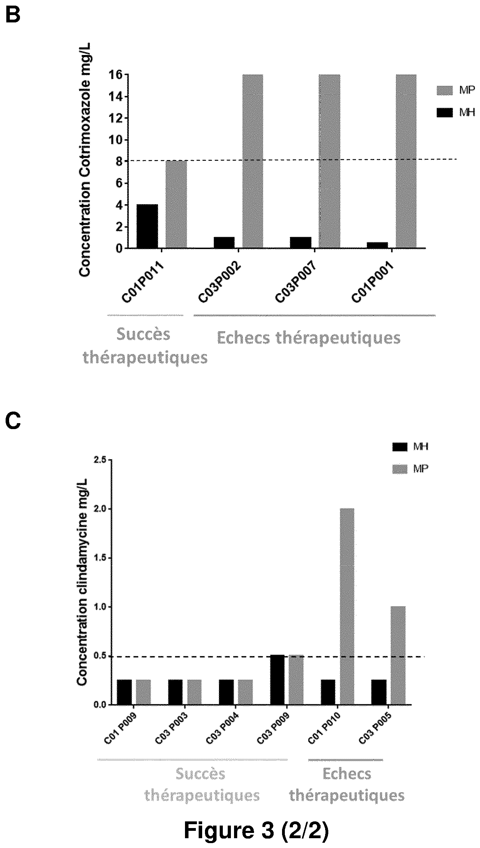

- La Figure 3 représente les résultats obtenus d'une détermination de sensibilité à des antibiotiques de souches cliniques de *S. aureus* issues d'infections de plaies du pied chez le diabétique. Les données cliniques, anonymisées, nécessaires à l'interprétation des résultats, consistaient en l'antibiothérapie donnée au patient, ainsi que la qualification par les médecins hospitaliers d'un succès ou échec thérapeutique. La définition de succès ou échec thérapeutique est définie comme suit. Le patient commence et suit l'antibiothérapie prescrite et un deuxième prélèvement microbiologique est effectué lors du dernier jour de traitement de la 1ère antibiothérapie (± 2 jours). Ce prélèvement est utilisé pour déterminer la présence/absence du pathogène ciblé par l'antibiothérapie, *S. aureus.* Si ce prélèvement a montré la persistance d'un *S. aureus,* l'antibiothérapie est en échec. A l'inverse, en cas d'absence de *S. aureus,* l'antibiothérapie est associée à un succès thérapeutique.
   Les antibiogrammes ont été réalisés classiquement sur une durée de 18h ± 2h en accord avec les recommandations de l'EUCAST. Une gamme de l'antibiotique à tester a été réalisée.
   La Figure 3A montre sous forme d'histogramme les résultats des CMI déduites d'antibiogrammes sur 11 souches testées en présence d'amoxicilline / clavulanate de potassium (commercialisé par exemple sous le nom d'Augmentin (marque déposée)). Sur la figure, l'ordonnée représente la concentration d'amoxicilline / clavulanate de potassium respectivement en mg/L et µg/L. L'abscisse correspond aux références des souches testées. Le milieu selon l'invention est désigné par MP et le milieu Mueller-Hinton est désigné par MH. Un seuil à 0,5 mg/L / 0,5 µg/L permettant de différencier les souches associées à un succès thérapeutiques des souches associées à un échec thérapeutique en fonction de leurs CMI dans un exemple de milieu selon l'invention (MP) a été établi. Cette valeur est représentée par une ligne horizontale en pointillés.
   La Figure 3B montre sous forme d'histogramme les résultats des CMI déduites d'antibiogrammes sur 4 souches testées en présence de cotrimoxazole (association de triméthoprime et sulfaméthoxazole, commercialisé par exemple sous le nom de Bactrim (marque déposée)). Un seuil à 8 mg/L permettant de différencier les souches associées à un succès thérapeutiques des souches associées à un échec thérapeutique en fonction de leurs CMI dans un exemple de milieu selon l'invention (MP) a été établi. Ce seuil est représenté par une ligne horizontale en pointillés. Sur la figure, l'ordonnée représente la concentration de cotrimoxazole en mg/L. L'abscisse correspond aux références des souches testées. Le milieu selon l'invention est désigné par MP et le milieu Mueller-Hinton est désigné par MH.
   La Figure 3C montre sous forme d'histogramme les résultats des CMI déduites d'antibiogrammes sur 6 souches testées en présence de clindamycine. Un seuil à 0,5 mg/L permettant de différencier les souches associées à un succès thérapeutiques des souches associées à un échec thérapeutique en fonction de leurs CMI dans un exemple de milieu selon l'invention (MP) a été établi. Ce seuil est représenté par une ligne horizontale en pointillés. Sur la figure, l'ordonnée représente la concentration de clindamycine en mg/L. L'abscisse correspond aux références des souches testées. Le milieu selon l'invention est désigné par MP et le milieu Mueller-Hinton est désigné par MH.

### EXEMPLES

### Exemple 1 : procédé de fabrication du milieu de culture et utilisations

Dans l'exemple ci-dessous, un exemple de milieu de culture selon l'invention est également désigné « milieu plaie ».

### MATÉRIELS ET MÉTHODES

### Préparation du milieu de culture

Un milieu Bolton (Bolton Broth, Base ; Fournisseur : Sigma Aldrich) a été stérilisé par autoclavage (120°C, 20 minutes). La composition du milieu Bolton était la suivante, pour 1 litre : Peptone de viande 10,0 g, Hydrolysat de lactalbumine 5,0 g, Extrait de levure 5,0 g, NaCI 5,0 g, Acide alpha-cétoglutarique 1,0 g, Pyruvate de sodium 0,5 g, Métabisulfite de sodium 0,5 g, Carbonate de sodium 0,6 g, Hémine 0,01 g, pH 7,2 - 7,4 à 25°C.

Après refroidissement, les autres constituants de l'exemple de milieu selon l'invention non autoclavés (sang, sérum, kératinocytes) ont été ajoutés tout en restant en surfusion dans le cas de milieu gélosé comme suit.

Le sang a été hémolysé en réalisant 4 cycles de congélation à -20°C / décongélation à température ambiante (25°C). Après le dernier cycle, le sang a été laissé à décanter à 4°C une nuit, puis le surnageant a été récupéré et le sang apporté dans le milieu à raison de 0,5% (v/v).

Le sérum utilisé a été assemblé à partir de 4 poches de 250 mL de 4 patients (sérotypages AB+ et AB-), a été décomplémenté par chauffage à 56°C pendant 30 minutes, puis a été stérilisé par filtration sur membrane 0,22 µm avant d'être ajouté dans le milieu à raison de 20% (v/v).

Les cellules de kératinocytes HaCaT, après dénombrement en cytométrie en flux (« FACS ») ont été lysées en réalisant 3 cycles de congélation dans l'azote liquide / décongélation à température ambiante, à savoir 25°C, puis leur lyse a été vérifiée au FACS avant de les ajouter au milieu à raison de 1.10⁶ cellules initiales/mL.

La composition du milieu obtenu était telle que décrit dans le Tableau 3 ou 4 :

**Tableau 3 : exemple de milieu de culture selon l'invention**

| **Composition du milieu** | **Proportion / concentration** |
|---|---|
| Milieu Bolton | 79,5% v/v |
| Sérum humain décomplémenté | 20% v/v |
| Sang humain hémolysé | 0,5% v/v |
| Débris de kératinocytes humains (lignée HaCaT) | 1.10⁶/mL |
| Tampon Hepes 100 mM, pH 8 (avec NaOH) | Hepes 100 mM - NaOH 1M |

Dans le Tableau 3, v/v signifie que le pourcentage est exprimé en volume / volume.

**Tableau 4 : exemple de composition de milieu de culture selon l'invention**

| **Composants pour 1L de milieu** | **Quantité** | **Concentration** (Pourcentage en poids par rapport au poids total de la composition) |
|---|---|---|
| Peptone de viande | 7,95 g | 0,795 % |
| Hydrolysat de lactalbumine | 3,98 g | 0,398 % |
| Extrait de levure | 3,98 g | 0,398 % |
| NaCl | 3,98 g | 0,398 % |
| Acide alpha-cétoglutarique | 0,79 g | 0,0795 % |
| Pyruvate de sodium | 0,39 g | 0,0397 % |
| Métabisulfite de sodium | 0,39 g | 0,0397 % |
| Carbonate de sodium | 0,48 g | 0,048 % |
| Hémine | 8 mg | 0,00795 % |
| Sang humain hémolysé | 500 µL | 5% |
| Sérum humain décomplémenté | 200 mL | 20% |
| Débris cellulaire de kératinocytes | 1x10⁹ débris | |
| HEPES ou bicarbonate de sodium 100mM, ajusté à pH 8,0 avec NaOH | 100 mL | |
| Eau déminéralisé ultra pure | QSP 1 L | |

Dans cet exemple et l'utilisation du milieu, la préparation du milieu se fait la veille de son utilisation, le milieu a été conservé à 4°C et le milieu a été conservé jusqu'à 4 jours.

Dans le cas d'utilisation de milieu gélosé, de l'agar a été ajouté au milieu Bolton avant stérilisation pour obtenir une concentration finale dans le milieu de 1% (w/v).

### Cinétique de croissance bactérienne

Afin d'étudier l'effet du milieu sur la croissance bactérienne, une étude de la cinétique de croissance de différentes bactéries (*S. aureus* ou *P. aeruginosa*) a été réalisée. Pour *S. aureus,* les 2 souches étaient : une souche sauvage de référence : *S. aureus* Newman et une souche clinique (SAC1) isolée à partir d'un prélèvement réalisé au cours d'une infection d'une plaie du pied chez un patient diabétique au CHU de Nîmes. Pour *P. aeruginosa,* les 2 souches étaient : une souche sauvage de référence (PAO1) et une souche clinique (PAC1) isolée à partir d'un prélèvement réalisé au cours d'une infection d'une plaie du pied chez un patient diabétique au CHU de Nîmes. Les souches SAC1 et PAC1 (pour respectivement *S. aureus* Clinique 1 et *P. aeruginosa* Clinique 1) ont été isolées à partir d'un même prélèvement réalisé chez un patient diabétique présentant une plaie infectée du pied.

Dans cet exemple les milieux utilisés étaient : un exemple de milieu selon l'invention (MP) tel qu'obtenu ci-dessus (Tableau 3) et un milieu « classique » de culture bactérienne : milieu BHI.

La composition du milieu BHI utilisé est la suivante :
∘ BHI (Brain Heart Infusion ; Bouillon Cœur Cervelle) pour 1 litre:
▪ Protéose-peptone 10,0 g
▪ Infusion de cervelle de veau 12,5 g
▪ Infusion de cœur de bœuf 5,0 g
▪ Glucose 2,0 g
▪ Chlorure de sodium 5,0 g
▪ Hydrogénophosphate de sodium 2,5 g
▪ pH = 7,2 - 7,6 à 25°C

La veille de la mesure de cinétique de croissance, les souches ont été cultivées sous agitation pendant une nuit, en respectant le volume d'oxygénation de 4/5 par rapport au volume de culture, dans le milieu qui a été utilisé pour la mesure de cinétique de croissance : milieu BHI ou exemple de milieu selon l'invention (MP). La cinétique de croissance a été réalisée dans un erlenmeyer de 250 mL contenant 50 mL du milieu testé. Une suspension bactérienne à DO 0,1 (600 nm) a été utilisée comme inoculum de départ.

Dans le cas de la co-culture (Figure 1C), les 2 inocula étaient identiques, de manière à partir d'un rapport 1:1 pour les 2 souches. Les cultures ont été mises sous agitation à 200 tours/min dans une étuve à 37°C. La cinétique de prélèvement a été réalisée aux temps suivants : 0, 30 minutes, 1H, 1H30, 2H, 2H30, 3H, 4H, 5H, 6H, 7H, 8H et 24H. A chaque temps, 200µL de la suspension bactérienne ont été prélevés et déposés dans une plaque 96 puits. Des dilutions successives, au 1/10ème ont été réalisées dans du Tampon Phosphate Salin 1X(« Phosphate Buffer Saline (PBS)) (30µL dans 270µL), et 100µL de chaque dilution ont été étalés sur gélose Luria-Bertani agar (LB agar). Les boites ont été laissées 24H à 37°C et les colonies ont été dénombrées. Les résultats sont exprimés en Log UFC/ml (Unités Formant Colonies/ml), en fonction du temps de lecture. Les résultats sont exprimés comme la moyenne des points obtenus, avec les écart-types, de 3 expériences indépendantes.

### Etude de l'adhésion et de la formation de biofilm par des bactéries S. aureus et P. aeruginosa

Pour *S. aureus,* les 2 souches montrées en exemple étaient une souche sauvage, de référence : *S. aureus* Newman et une souche clinique (SAC1) isolée à partir d'un prélèvement réalisé au cours d'une infection de plaie du pied chez le diabétique au CHU de Nîmes.

Pour *P. aeruginosa,* les 2 souches montrées en exemple étaient une souche sauvage, de référence (PAO1) et une souche clinique (PAC1) isolée à partir d'un prélèvement réalisé au cours d'une infection de plaie du pied chez le diabétique au CHU de Nîmes. L'isolement bactérien à partir du prélèvement de plaie réalisé chez le patient a montré la présence de *S. aureus* et *P. aeruginosa* qui ont été utilisés dans ces exemples sous les noms de SAC1 et PAC1 (pour respectivement *S. aureus* Clinique 1 et *P. aeruginosa* Clinique 1).

La veille de la mesure de l'adhésion et de la formation du biofilm, les souches ont été cultivées pendant une nuit sur le milieu gélosé correspondant au bouillon (BHI ou un exemple de milieu selon l'invention tel que décrit dans le Tableau 4 ci-dessus) qui a été utilisé pour la mesure de l'adhésion et de la formation de biofilm (BHI ou milieu plaie, MP). La mesure de l'adhésion et de la formation de biofilm a été réalisée dans les puits de microplaque à raison de 200µL de milieu par puits, avec un inoculum initial correspondant à 0,1 DO 600nm. Dans le cas de cocultures (Fig. 3B), l'inoculum initial correspondait également à 0,1 DO 600nm, et 100µL de chaque souche étaient ajoutés dans chaque puits. Les cultures ont été mises dans une étuve à 37°C, avec 5% de CO₂, pendant 24H. Le milieu était ensuite éliminé par aspiration délicate, et les puits ont été rincés 3 fois avec 200µL de PBS 1X. De nouveau, 200µL de PBS ont été ajoutés et le fond des puits a été gratté avec un cône afin de détacher les bactéries ayant adhérées à la plaque. La microplaque a été ensuite recouverte d'un film adhésif et elle a été soniquée pendant 10 minutes, dans un sonicateur, à une fréquence de 40 KHz. Des dilutions successives, au 1/10ème ont été réalisées dans du PBS 1X (20µL dans 180µL) et 100µL ont été étalés sur LB agar. Les boites ont été laissées 24H à 37°C et les colonies ont été dénombrées. Les résultats sont exprimés en Log UFC/ml (Unités Formant Colonies/mL), en fonction du temps de lecture.

### Étude de de sensibilité aux antibiotiques de bactéries S. aureus

Les tests de sensibilité ont été réalisés sur des souches de *S. aureus* issues de prélèvements de plaies du pied infectées chez des patients diabétiques. Les tests de sensibilité aux antibiotiques ont été réalisés en milieu Muller-Hinton (MH) ou dans un exemple de milieu selon l'invention (MP) tel que décrit dans le Tableau 4 ci-dessus. Pour les 2 milieux les conditions d'inoculum (0,0002 DO 600nm, soit 2.10⁵ UFC/mL) et d'incubation (18H à 37°C) étaient identiques. Les incubations ont été réalisées dans des microplaques 96 puits. La croissance bactérienne a été déterminée par mesure de la densité optique à 600nm. La valeur de CMI a été déterminée comme la plus faible concentration inhibant la croissance bactérienne, correspondant au puits donnant une DO 600nm égale à celle du contrôle (milieu sans bactérie) ±3 écart-types. Les données concernant les antibiotiques prescrits et les données cliniques (succès ou échec thérapeutique) des patients ont été obtenues de façon anonymisée. Un succès ou un échec a été défini comme suit : à l'issue de la première ligne d'antibiothérapie +/- 2 jours, dont la durée peut varier selon l'antibiotique prescrit, un prélèvement microbiologique est réalisé. L'absence de *S. aureus* constitue un succès thérapeutique, alors qu'au contraire, la présence de *S. aureus* constitue un échec thérapeutique. Le Tableau 5 résume les résultats de CMI des souches dans les milieux MH et MP (exemple de milieu selon l'invention (MP)). Les antibiotiques testés ayant été prescrits pour le traitement de la pathologie liée à ces souches, les souches avaient été définies comme sensibles aux antibiotiques par les méthodes d'antibiogramme conventionnelle. Le tableau qualifie les souches R (résistante) ou S (sensible) en fonction de seuils permettant de différencier les souches associées à un succès thérapeutiques des souches associées à un échec thérapeutique en fonction de leurs CMI mesurées dans un exemple de milieu selon l'invention (MP). Ce seuil a été fixé à 0,5 mg/L / 0,5 µg/L pour l'association amoxicilline / clavulanate de potassium, 8 mg/L pour le cotrimoxazole, et 0,5 mg/L pour la clindamycine. La Figure 3 est une représentation sous forme d'histogrammes des résultats obtenus.
Les antibiotiques prescrits aux patients ont été testés : l'association amoxicilline / clavulanate de potassium (commercialisé par exemple sous le nom de marque Augmentin (marque déposée)), le cotrimoxazole, association de triméthoprime et de sulfamethoxazole (commercialisé par exemple sous le nom de marque Bactrim (marque déposée)), et la clindamycine (commercialisée par exemple sous le nom de marque Dalacine (marque déposée)). Ces 3 préparations antibiotiques appartiennent à 3 classes différentes, de par leurs mécanismes d'action.

### Résultats

### Cinétique de croissance bactérienne

La Figure 1 représente les résultats de croissance de bactéries en fonction du milieu formalisé sous la forme de courbes. Sur cette figure, les ordonnées correspondent au logarithme d'unités formant colonie par millilitre de milieu de culture (Log UFC/mL) et les abscisses le temps en heure (H). La Figure 1A montre les courbes de croissance (« fitness ») de 2 souches de *S. aureus* dans le milieu (BHI) (ronds noirs) ou un exemple de milieu selon l'invention (MP) (carrés gris). Tel que démontré sur la Figure 1A, pour la souche de référence *S. aureus* Newman (Newman) et pour la souche *S. aureus* clinique issue d'un prélèvement de plaie infectée du pied chez un patient diabétique *S. aureus* SAC1 (SAC1), la croissance est plus rapide en milieu BHI (ronds noirs) que dans l'exemple de milieu selon l'invention (carrés gris). En outre, il apparait clairement que le plateau de croissance est atteint plus rapidement pour la culture avec un exemple de milieu selon l'invention, pour une charge bactérienne plus faible. La Figure 1B correspond à des courbes de croissance (« fitness ») de 2 souches de *P. aeruginosa* dans le milieu (BHI) (ronds noirs) ou un exemple de milieu selon l'invention (MP) (carrés gris). Tel que démontré sur cette figure, la croissance de la souche de référence (PAO1) et la croissance de la souche clinique issue du couple 1 (PAC1) sont similaires quel que soit le milieu de culture utilisé. Il est à noter qu'une différence est observée concernant l'atteinte du plateau de croissance qui est plus rapide pour une charge bactérienne plus faible lorsque les souches ont été cultivées dans un exemple de milieu selon l'invention. La Figure 1C représente les résultats correspondant aux courbes de croissance (« fitness ») des 2 souches cliniques issues de la même infection (couple de souches), *S. aureus* SAC1 et *P. aeruginosa* PAC1, dans le milieu (BHI) (ronds noirs) ou un exemple de milieu selon l'invention (MP) (carrés gris). Tel que démontré dans cette figure, en coculture la croissance de la souche clinique *S. aureus* SAC1 était similaire quel que soit le milieu utilisé (BHI ou MP) et était proche de la croissance de la souche cultivée en monoculture en BHI (Fig. 1A). Toutefois, il est à noter qu'aucun plateau de croissance n'a été observé, au contraire de la condition en monoculture. De la même façon, il apparaît que, dans ces conditions de coculture, la croissance de la souche clinique *P. aeruginosa* PAC1 était similaire quel que soit le milieu utilisé (BHI ou MP), comme lors de la monoculture (Fig. 1B). Toutefois, il est à noter qu'aucun plateau de croissance n'a été observé, aux temps de lecture, au contraire de la condition en monoculture.

Ces résultats démontrent clairement qu'un milieu selon invention permet avantageusement de cultiver et d'obtenir une croissance de microorganismes, en particulier de bactéries, en monoculture ou en coculture. Ces résultats démontrent également clairement qu'un milieu selon l'invention permet avantageusement une culture simultanée (coculture) de différentes souches et/ou types de microorganismes, par exemple différentes souches bactériennes. Ces résultats démontrent également avantageusement les possibles différences de comportement de souches entre un milieu selon l'invention, mimant leur environnement d'origine, et un milieu de culture microbiologique utilisé pour la croissance de nombreux types de microorganismes, quelle que soit leur origine.

Les résultats précités démontrent également clairement que le milieu selon l'invention permet avantageusement une culture simultanée (coculture) de différents types de microorganismes, par exemple de bactéries à Gram négatif et de bactéries à Gram positif. En outre, cet exemple démontre clairement qu'un milieu selon l'invention permet avantageusement une culture et une croissance de bactéries isolées le plus fréquemment dans les plaies chroniques, *S. aureus* (cocci à Gram positif) et *P. aeruginosa* (bacille à Gram négatif). Les résultats démontrent également clairement que le milieu selon l'invention permet avantageusement de modéliser et/ou de mimer les conditions environnementales, par exemple présentes dans des plaies, et avantageusement de révéler les différences de croissances et/ou comportement des microorganismes, par exemple des bactéries, dans cet environnement. Les résultats précités démontrent en outre clairement qu'un milieu, selon l'invention, permet avantageusement, notamment lors de la coculture de différentes espèces/souches de bactéries, leurs interactions, par exemple de manière similaire à l'environnement dont elles sont issues, par exemple de plaies, et permet de révéler un éventuel comportement et/ou évènement différentiel par rapport à leur monoculture.

### Étude de l'adhésion et de la formation de biofilm par des bactéries S. aureus et P. aeruginosa

La Figure 2 représente les résultats d'une étude de la formation de biofilm, la Figure 2A correspond aux résultats du dénombrement des bactéries, cultivées en monoculture, adhérées au fond du puits, à 24H. Les bactéries ont été cultivées pendant 24H dans les puits de microplaques 96 puits et les bactéries adhérées ont été dénombrées par étalement de dilutions sur milieu gélosé après élimination des bactéries non adhérées et détachement des bactéries adhérées. Pour les 3 souches étudiées, *S. aureus* Newman et SAC1, *P. aeruginosa* PAO1, le nombre de bactéries était significativement plus important (p value <0,01 : **) avec le milieu selon l'invention qu'avec le milieu BHI. En particulier, le nombre de bactéries était supérieur à environ une unité Log dans le milieu selon l'invention, soit 10 fois supérieurs. Une différence significative a été également observée pour la souche PAC1 avec une croissance supérieure dans le milieu selon l'invention. Ces résultats démontrent que dans l'exemple de milieu selon l'invention, la quantité de biofilm formée, ou biomasse, déterminée par le nombre de bactéries adhérées, est plus importante dans le milieu selon l'invention que dans le milieu BHI.

La Figure 2B correspond aux résultats du dénombrement des bactéries obtenues, cultivées en coculture, adhérées au fond du puits, à 24H. Une coculture des bactéries a été effectuée pendant 24H, dans des puits de microplaques 96 puits, soit les souches de référence *S. aureus* Newman et *P. aeruginosa* PAO1 (PAO1/Newman), soit les souches cliniques, issues d'une même plaie chronique de pied diabétique, *S. aureus* SAC1 et *P. aeruginosa* PAC1 (PAC1/SAC1) dans un milieu selon l'invention (bâtons gris) ou dans un milieu BHI (bâtons noirs). Les bactéries adhérées ont été dénombrées par étalement de dilutions sur milieu gélosé après élimination des bactéries non adhérées et détachement des bactéries adhérées. Pour chaque condition de coculture, l'ensemble des bactéries a été dénombré et représenté sur le graphe. Comme pour les monocultures, l'adhésion des souches était plus forte avec le milieu selon l'invention qu'en milieu BHI. La différence était de 2 Log pour les souches de références, et de 3 Log pour les souches cliniques. Ainsi, la différence est hautement significative (p-value <0,001 : ***) dans le cas des souches cliniques, et significative (p-value <0,01 : **) pour les souches de référence. Sur les géloses ayant servi au dénombrement, la morphologie des colonies de *S. aureus* et *P. aeruginosa* étant différente, un dénombrement de chaque espèce a été réalisé et a montré une répartition de 60% de *P. aeruginosa* et 40% de *S. aureus.*

### Étude de sensibilité aux antibiotiques des bactéries de l'espèce S. aureus

L'étude comparative de sensibilité aux antibiotiques de souches de *S. aureus* associées à des infections de plaies du pied chez le diabétique a été réalisée sur un panel de souches isolées au CHU de Nîmes et pour lesquelles les données cliniques associées à l'antibiothérapie (succès ou échec thérapeutique) étaient disponibles.

Les résultats obtenus sont représentés dans la Figure 3A (amoxicilline / clavulanate de potassium), 3B (cotrimoxazole) et 3C (clindamycine) et la synthèse des résultats est représentée dans les Tableaux 5A, 5B et 5C.

### Tableau 5

**Tableau 5A : antibiogrammes des souches traitées par amoxicilline/clavulanate de potassium**

| Souche | CMI en milieu MH | Classification (S) ou (R) en MH | CMI en milieu plaie MP | Classification (S) ou (R) en MP | Succès ou échec thérapeutique |
|---|---|---|---|---|---|
| C01P012 | 0,25 | (S) | 0,25 | (S) | Succès |
| C03P001 | 0,25 | (S) | 0,5 | (S) | Succès |
| C04P003 | 0,25 | (S) | 0,25 | (S) | Succès |
| C04P005i | 0,5 | (S) | 0,5 | (S) | Succès |
| C04P005f | 0,5 | (S) | 0,5 | (S) | Succès |
| C03P008 | 0,5 | (S) | 1 | (R) | Echec |
| C04P007 | 0,25 | (S) | 1 | (R) | Echec |
| C06P001 i | 0,25 | (S) | 4 | (R) | Echec |
| C06P001f | 0,25 | (S) | 2 | (R) | Echec |
| C06P003i | 0,25 | (S) | 2 | (R) | Echec |
| C06P003f | 0,25 | (S) | 1 | (R) | Echec |

**Tableau 5B : antibiogrammes des souches traitées par cotrimoxazole**

| Souche | CMI en milieu MH | Classification (S) ou (R) en MH | CMI en milieu plaie MP | Classification (S) ou (R) en MP | Succès ou échec thérapeutiqu e |
|---|---|---|---|---|---|
| C01P0 11 | 4 | (S) | 8 | (S) | Succès |
| C03P0 02 | 1 | (S) | 16 | (R) | Echec |
| C03P0 07 | 1 | (S) | 16 | (R) | Echec |
| C01P0 01 | 0,5 | (S) | 16 | (R) | Echec |

**Tableau 5C : antibiogrammes des souches traitées par clindamycine**

| Souche | CMI en milieu MH | Classification (S) ou (R) en MH | CMI en milieu plaie MP | Classification (S) ou (R) en MP | Succès ou échec thérapeutique |
|---|---|---|---|---|---|
| C01P009 | 0 ,25 | (S) | 0,25 | (S) | Succès |
| C03P003 | 0,25 | (S) | 0,25 | (S) | Succès |
| C03P004 | 0,25 | (S) | 0,25 | (S) | Succès |
| C03P009 | 0,5 | (S) | 0,5 | (S) | Succès |
| C01P010 | 0,25 | (S) | 2 | (R) | Echec |
| C03P005 | 0,25 | (S) | 1 | (R) | Echec |

La détermination de la sensibilité de *S*. *aureus* à l'amoxicilline / clavulanate de potassium a été réalisée sur 5 souches associées à un succès thérapeutique, et 6 souches associées à un échec thérapeutique. Tel que représenté sur le Tableau 5A, ainsi que la Figure 3A, les résultats démontrent que dans le milieu MH, les CMI mesurées étaient comprises entre 0,25 mg/L / 0,25 µg/L et 0,5 mg/L / 0,5 µg/L pour toutes les souches, qu'elles soient associées à un succès thérapeutique ou à un échec thérapeutique. A l'inverse, les résultats démontrent que dans un exemple de milieu selon l'invention (MP), les CMI mesurées étaient comprises entre 0,25 mg/L / 0,25 µg/L et 0,5 mg/L 0,5 µg/L pour les souches associées à un succès thérapeutique, et comprises entre 1 mg/L / 1 µg/L et 4 mg/L / 4 µg/L pour les souches associées à un échec thérapeutique. Ces résultats démontrent clairement que l'exemple de milieu selon l'invention permet de déterminer une CMI distincte en fonction du succès ou de l'échec thérapeutique. Tel que démontré l'utilisation du milieu MH, il n'est pas possible de différencier en fonction de la CMI mesurée les souches associées à un succès thérapeutique des souches associées à un échec thérapeutique. A l'inverse, avec l'utilisation d'un exemple de milieu selon l'invention (MP), il est possible de différencier les souches associées à un succès thérapeutique des souches associées à un échec thérapeutique en fonction de leur CMI, par exemple via la définition d'un seuil à 0,5 mg/L / 0,5 µg/L (représenté par une ligne horizontale en pointillés dans la Figure 3A). Tel que démontré, les 5 souches associées à un succès thérapeutique ont une valeur de CMI mesurés dans l'exemple de milieu selon l'invention (MP), inférieure ou égale au seuil et ont été et peuvent être classifiées comme sensibles (noté S dans le tableau 5A). Les 6 souches associées à un échec thérapeutique ont une valeur de CMI mesurée dans l'exemple de milieu selon l'invention (MP) supérieure au seuil et peuvent être classifiées comme résistantes (noté R dans le tableau 5A).

Tel que démontré ci-dessus, la présente invention permet avantageusement de déterminer de manière plus fiable et plus précise la sensibilité de microorganismes présents dans des plaies à des antibiotiques et d'éviter un éventuel échec du traitement antibiotique ou d'augmenter les chances de succès du traitement, par exemple antibiotique, par exemple le traitement d'une plaie infectée, par exemple avec la bactérie *S*. *aureus,* avec de l'amoxicilline / clavulanate de potassium.

La détermination de la sensibilité de *S*. *aureus* au cotrimoxazole a été réalisée sur 1 souche associée à un succès thérapeutique, et 3 souches associées à un échec thérapeutique. Les résultats obtenus sont représentés dans le tableau 5 B et sur la Figure 3B. Tel que représenté sur le Tableau 5B, ainsi que la Figure 3B, les résultats démontrent que dans le milieu MH, les CMI mesurées étaient comprises entre 0,5 mg/L et 4 mg/L pour toutes les souches. Etonnamment, la CMI de la souche associée à un succès thérapeutique (4 mg/L) était plus importante que les CMI des souches associées à un échec thérapeutique (0,5 mg/L à 1 mg/L). A l'inverse, les résultats démontrent que dans un exemple de milieu selon l'invention (MP), la CMI mesurée pour la souche associée à un succès thérapeutique (8 mg/L) était inférieure aux CMI des souches associées à un échec thérapeutique (16 mg/L). Tel que démontré, les CMI mesurées dans un exemple de milieu selon l'invention permettent clairement de distinguer le succès thérapeutique de l'échec thérapeutique. De plus l'exemple de milieu selon l'invention (MP) permet avantageusement de définir et/ou déterminer un seuil, par exemple à 8 mg/L (représenté par une ligne horizontale en pointillés dans la Figure 3B) permettant de différencier les souches associées à un succès thérapeutique des souches associées à un échec thérapeutique en fonction de leur CMI. Tel que démontré, la détermination de la CMI avec un exemple de milieu selon l'invention pour une souche associée à un succès thérapeutique a une valeur de CMI inférieure ou égale à celle de la CMI pour les souches associées à un échec et avantageusement inférieur au seuil et peut être classifiée comme sensible (noté S dans le tableau 5B). Les 3 souches associées à un échec thérapeutique ont une valeur de CMI mesurée dans l'exemple de milieu selon l'invention supérieure au seuil et peuvent être classifiées comme résistantes (noté R dans le tableau 5B).

Tel que démontré ci-dessus, la présente invention permet avantageusement de déterminer de manière plus fiable et plus précise la sensibilité de microorganismes présents dans des plaies à des antibiotiques et d'éviter un éventuel échec du traitement antibiotique ou d'augmenter les chances de succès du traitement, par exemple antibiotique, par exemple le traitement d'une plaie infectée, par exemple avec la bactérie *S*. *aureus,* avec du cotrimoxazole.

La détermination de la sensibilité de *S*. *aureus* à la clindamycine a été réalisée sur 4 souches associées à un succès thérapeutique, et 2 souches associées à un échec thérapeutique. Les résultats obtenus sont représentés dans le tableau 5 C et sur la Figure 3C. Tel que représenté sur le Tableau 5C, ainsi que la Figure 3C, les résultats démontrent que dans le milieu MH, les CMI mesurées étaient comprises entre 0,25 mg/L et 0,5 mg/L pour toutes les souches, qu'elles soient associées à un succès thérapeutique ou à un échec thérapeutique. A l'inverse, les résultats démontrent que dans un milieu plaie, exemple de milieu selon l'invention (MP), les CMI mesurées étaient comprises entre 0,25 mg/L et 0,5 mg/L pour les souches associées à un succès thérapeutique, et comprises entre 1 mg/L et 2 mg/L pour les souches associées à un échec thérapeutique. Tel que démontré, les CMI mesurées dans un exemple de milieu selon l'invention permettent clairement de distinguer un succès thérapeutique d'un échec thérapeutique. Avec l'utilisation du milieu MH, il n'est pas possible de différencier, en fonction de la valeur de CMI mesurée, les souches associées à un succès thérapeutique des souches associées à un échec thérapeutique. En outre, avec l'utilisation de l'exemple de milieu selon l'invention (MP), il est avantageusement possible de définir un seuil pour la CMI, par exemple à 0,5 mg/L (représenté par une ligne horizontale en pointillés dans la Figure 3C) permettant de différencier les souches associées à un succès thérapeutique des souches associées à un échec thérapeutique en fonction de la CMI mesurée dans un milieu plaie, exemple de milieu selon l'invention (MP). Tel que démontré, les 4 souches associées à un succès thérapeutique ont une valeur de CMI mesurée dans un milieu plaie, exemple de milieu selon l'invention (MP) inférieure ou égale au seuil déterminé et peuvent être classifiées comme sensibles (noté S dans le tableau 5C). Les 2 souches associées à un échec thérapeutique ont une valeur de CMI mesurée dans un milieu plaie, exemple de milieu selon l'invention (MP), supérieure au seuil et peuvent être classifiées comme résistantes (noté R dans le tableau 5C).

Tel que démontré ci-dessus, la présente invention permet avantageusement de déterminer de manière plus fiable et plus précise la sensibilité de microorganismes présents dans des plaies à des antibiotiques et d'éviter un éventuel échec du traitement antibiotique ou d'augmenter les chances de succès du traitement, par exemple antibiotique, par exemple le traitement d'une plaie infectée, par exemple avec la bactérie *S*. *aureus,* avec de la clindamycine.

Ces exemples démontrent clairement qu'un milieu selon l'invention permet avantageusement de cultiver des microorganismes et/ou de mesurer la croissance de microorganismes.

Ces exemples démontrent également clairement qu'un milieu selon l'invention permet une coculture de plusieurs espèces de microorganismes, en particulier de bactéries. En outre, cet exemple démontre clairement que le milieu de culture selon l'invention permet avantageusement une coculture de *Staphylococcus aureus* et *Pseudomonas aeruginosa,* sans être restreint à ces 2 types de bactéries, qui correspondent et représentent les bactéries les plus fréquemment isolées dans une plaie, en particulier une plaie chronique.

Ces exemples démontrent de plus que le milieu selon l'invention permet notamment une coculture de bactéries à Gram positif et à Gram négatif.

Ces exemples démontrent en outre qu'un milieu selon l'invention permet avantageusement de reproduire / mimer des facteurs environnementaux et/ou des conditions environnementales dans lesquels les microorganismes se développent.

Ces exemples démontrent également clairement qu'un milieu selon l'invention permet avantageusement une coculture ou non de microorganismes, notamment dans différentes conditions, par exemple planctonique ou biofilm, et/ou de tester et/ou étudier les caractéristiques de la croissance de microorganismes, par exemple de bactéries quel que soit le mode de culture.

Ces exemples démontrent également qu'un milieu selon l'invention permet avantageusement de reproduire et/ou mimer l'environnement d'une plaie chronique humaine et permet avantageusement d'évaluer la croissance de microorganismes, en monoculture ou coculture, avantageusement selon une réalité de l'infection de la plaie considérée, la formation de biofilm, et la susceptibilité aux antimicrobiens (détermination de CMI).

Ces exemples démontrent en outre qu'un milieu selon l'invention permet avantageusement de déterminer de manière plus fiable et plus précise la sensibilité de microorganismes, en particulier présents dans des plaies, à des composés, par exemple des antibiotiques et avantageusement d'éviter un éventuel échec d'un traitement et/ou d'augmenter les chances de succès de ce traitement.

### Listes des références

**1.** Donlan, R. M. and J. W. Costerton. (2002). Biofilms: survival mechanisms of clinically relevant microorganisms. Clin Microbiol Rev. 2002 ; 15(2):167-193. Reproduced with permission from American Society for Microbiology.
**2.** Høiby N. A personal history of research on microbial biofilms and biofilm infections. Pathog Dis. 2014 Apr; 70(3):205-11.
**3.** Liu Y. (1999) Domestic sewage treatment by an Ultra-Compact Biofilm reactor. MEng Thesis , National University of Singapore, Singapore
**4.** Potera C. Forging a link between biofilms and disease. Science. 1999 Mar 19; 283(5409):1837, 1839.
**5.** Reid G, Howard J, Gann BS. Can bacterial interference prevent infection? Trends Microbiol. 2001 Sep; 9(9):424-8.
**6.** Gristina AG, Costerton JW. Bacterial adherence to biomaterials and tissue. The significance of its role in clinical sepsis. J Bone Joint Surg Am. 1985 Feb; 67 (2):264-73.
**7.** Newman LG, Waller J, Palestra CJ, Schwartz M, Klein MJ, Hermann G, Harrington E, Harrington M, Roman SH, Saguaro-Green A. Unsuspected osteomyelitis in diabetic foot ulcers. Diagnosis and monitoring by leukocyte scanning with indium in 111 oxyquinoline. JAMA. 1991 Sep 4;266(9):1246-51.
**8.** James GA, Swagger E, Wolcott R, Pulcini Ed, Secor P, Sestrich J, Costerton JW, Stewart PS. Biofilms in chronic wounds. Wound Repair Regen. 2008 Jan-Feb;16(1):37-44.
**9.** Metcalf D. & Bowler P. Biofilm delays wound healing: A review of the evidence. Burns Trauma. 2013; 1. 5-12. 10.4103/2321-3868.113329.
**10.** Hinman CD, Maibach H. Effect of air exposure and occlusion on experimental human skin wounds, Nature 1963; 200:377-478.
**11.** Cutting KF. Wound exudate: composition and functions. Br J Community Nurs. 2003;8(9 Suppl):suppl 4-9.
**12.** World Health Organization. (1961). Standardization of Methods for Conducting Microbic Sensitivity Tests. Second Report of the Expert Committee on Antibiotics. WHO Technical Report Series, No. 210. WHO, Geneva.
**13.** Sawer IK, Berry MI, Ford JL. Effect of medium composition, agitation and the presence of EDTA on the antimicrobial activity of cryptolepine. Lett Applied Microbiol. 1997; 25: 207-211.
**14.** Ersoy SC, Heithoff DM, Barnes 5th L, Tripp GK, House JK, Marth JD, Smith JW, Mahan MJ. Correcting a Fundamental Flaw in the Paradigm for Antimicrobial Susceptibility Testing. EBioMedicine. 2017; 20:173-181.
**15.** Sun Y, Dowd SE, Smith E, Rhoads DD, Wolcott RD. In vitro multispecies Lubbock chronic wound biofilm model. Wound Repair Regen. 2008 Nov-Dec;16 (6):805-13. doi: 10.1111/j.1524-475X.2008.00434.x.
**16.** Pitts B, Hamilton MA, Zelver N, Stewart PS. A microtiter-plate screening method for biofilm disinfection and removal. J Microbiol Methods. 2003; 54(2): 269-276.
**17.** DeLeon S, Clinton A, Fowler H, Everett J, Horswill AR, Rumbaugh KP. Synergistic interactions of Pseudomonas aeruginosa and Staphylococcus aureus in an in vitro wound model. Infect Immun. 2014; 82 (11): 4718-28. doi: 10.1128/IAI.02198-14.
**18.** Bandyopadhyay B, Fan J, Guan S, Li Y, Chen M, Woodley DT, Li W. A "traffic control" role for TGFβ3: orchestrating dermal and epidermal cell motility during wound healing. J Cell Biol. 2006; 172(7): 1093-1105.
**19.** Loesche M, Gardner SE, Kalan L, Horwinski J, Zheng Q, Hodkinson BP, Tyldsley AS, Franciscus CL, Hillis SL, Mehta S, Margolis DJ, Grice EA. Temporal stability in chronic wound microbiota is associated with poor healing. J Invest Dermatol. 2017; 137(1): 237-244.
**20.** Jneid J, Cassir N, Schuldiner S, Jourdan N, Sotto A, Lavigne JP, La Scola B. Exploring the Microbiota of Diabetic Foot Infections With Culturomics. Front Cell Infect Microbiol. 2018; 8: 282. doi: 10.3389/fcimb.2018.00282.
**21.** Kadam S, Madhusoodhanan V, Bhide D, Ugale R, Tikhole U, Kaushik KS. Milieu matters : An in vitro wound milieu to recapitulate key features of, and probe new insights into, polymicrobial biofilms. bioRxiv preprint doi: https://doi.org/10.1101/2021.01.07.425734
**22.** Ölander M, Handin N, Artursson P. Image-Based Quantification of Cell Debris as a Measure of Apoptosis. Anal Chem. 2019 May 7;91 (9):5548-5552. doi: 10.1021/acs.analchem.9b01243. Epub 2019 Apr 25. PMID: 31001971.
**23.** Smucker and Pfister. Liquid Nitrogen Cryo-Impacting: a New Concept for Cell Disruption. Applied Microb. 1975; 30: 445-449.
**24.** Strobel, H. J. (2009). Basic laboratory culture methods for anaerobic bacteria. Biofuels, 247-261.
**25.** Ferrara-Guerrero, M. J., & Bianchi, A. (1989). Comparison of culture methods for enumeration of microaerophilic bacteria in marine sediments. Research in microbiology, 140(3), 255-261.
**26.** Richards JC, Jason AC, Hobbs G, Gibson DM, Christie RH. Electronic measurement of bacterial growth. J Phys E. 1978; 11 (6): 560-568.
**27.** Panikov NS. Microbial growth kinetics (Panikov, 1995, Trends in Microbiology, Vol. 4, Issue 8, p335.

## Revendications

1. Milieu de culture de microorganismes comprenant un hydrolysat enzymatique de protéines, de préférence un hydrolysat pepsique de protéines, un hydrolysat de protéines, un extrait de levure, du sérum de mammifère, de préférence du sérum humain, du sang de mammifère, des débris cellulaires, au moins un sel, dans lequel le pH du milieu est compris entre 7,5 et 8,5, de préférence égale à 8.

2. Milieu de culture de microorganismes selon la revendication 1, dans lequel l'hydrolysat enzymatique de protéines est un hydrolysat pepsique de protéines est choisi parmi de la peptone d'origine animale, de la peptone d'origine laitière, de la peptone d'origine végétale et/ou de la peptone mycélienne.

3. Milieu de culture de microorganismes selon la revendication 1 ou 2, la concentration de l'hydrolysat enzymatique de protéines est comprise de 0,75 à 0,85 % en poids, de préférence égale à 0,795 % en poids par rapport au poids total du milieu de culture.

4. Milieu de culture de microorganismes selon l'une quelconque des revendications précédentes dans lequel l'hydrolysat de protéines est choisi parmi de l'hydrolysat de lactalbumine, de lactoglobuline, d'albumine.

5. Milieu de culture de microorganismes selon l'une quelconque des revendications précédentes dans lequel la concentration de l'hydrolysat de protéines est comprise de 0,395 à 0,42 % en poids, de préférence égale à 0,398 % en poids par rapport au poids total du milieu de culture.

6. Milieu de culture de microorganismes selon l'une quelconque des revendications précédentes, dans lequel le sérum de mammifère est du sérum humain.

7. Milieu de culture de microorganismes selon l'une quelconque des revendications précédentes dans lequel la concentration de sérum de mammifère est comprise de 17 à 23 % en volume, de préférence égale à 20 % en volume par rapport au volume total du milieu de culture.

8. Milieu de culture de microorganismes selon l'une quelconque des revendications précédentes, dans lequel les débris cellulaires sont choisi parmi des débris de kératinocytes, de mélanocytes, de fibroblastes, d'histiocytes, de mastocytes ou de macrophages, ou un mélange de ceux-ci.

9. Milieu de culture de microorganismes selon la revendication 8, dans lequel la concentration de débris cellulaires est compris de 1x10⁸ à 1x10¹⁰ débris par litre, de préférence égale à 1x10⁹ débris par litre de milieu de culture.

10. Milieu de culture de microorganismes selon l'une quelconque des revendications précédentes, dans lequel le sang de mammifère est choisi dans le groupe comprenant du sang humain ou animal.

11. Milieu de culture de microorganismes selon l'une quelconque des revendications précédentes dans lequel la concentration de sang de mammifère est comprise de 0,5 à 1 % en volume, de préférence égale à 0,5 % en volume par rapport au volume total du milieu de culture.

12. Milieu de culture de microorganismes selon l'une quelconque des revendications précédentes, dans lequel la concentration de l'extrait de levure dans le milieu de culture est comprise de de 0,395 à 0,42 % en poids, de préférence égale à 0,398 % en poids par rapport au volume total du milieu de culture.

13. Milieu de culture de microorganismes selon l'une quelconque des revendications précédentes, ledit milieu comprenant un tampon choisi dans le groupe comprenant du tampon Hepes et du tampon bicarbonate.

14. Milieu de culture de microorganismes selon l'une quelconque des revendications précédentes, ledit milieu comprenant de l'agar-agar compris entre 0,8 et 2,0% en poids/volume, préférentiellement 1%.

15. Milieu de culture de microorganismes selon l'une quelconque des revendications précédentes dans lequel le sel est choisi dans le groupe comprenant le NaCl, KCl, CuCl₂, ZnCl₂, de préférence NaCl

16. Utilisation du milieu de culture selon l'une quelconque des revendications 1 à 16 dans un procédé de culture de microorganismes et/ou de détermination de la croissance et/ou cinétique de croissance de microorganismes.

17. Utilisation du milieu de culture selon l'une quelconque des revendications 1 à 16 dans un procédé de détermination de la sensibilité de microorganismes à au moins un composé test.

18. Utilisation du milieu de culture selon l'une quelconque des revendications 1 à 16 dans un procédé de culture d'une pluralité de microorganismes.

19. Utilisation du milieu de culture selon l'une quelconque des revendications 1 à 16 dans un procédé de détermination et/ou d'identification de la formation de biofilm par des microorganismes.

20. Procédé de fabrication d'un milieu de culture de microorganismes selon l'une quelconque des revendications 1 à 16 comprenant les étapes de :
**a.** hémolyse de sang de mammifère et décantage dudit sang hémolysé,
**b.** décomplémentation du sérum de mammifère et stérilisation du sérum décomplémenté,
**c.** mélange et stérilisation par chauffage ou filtration d'une solution aqueuse comprenant d'un hydrolysat enzymatique de protéines, de préférence un hydrolysat pepsique de protéines, d'un hydrolysat de protéines et d'un extrait de levure,
**d.** refroidissement du mélange stérile obtenu à l'étape c,
**e.** mélange dudit sang hémolysé obtenu à l'étape a, dudit sérum décomplémenté obtenu à l'étape b, du mélange stérile refroidit obtenu à l'étape d et de débris cellulaires, et obtention du milieu de culture.
